(19) Europäisches Patentamt
European Patent Office
Office européen des brevets

(11) **EP 3 834 839 B1**

(12) **EUROPEAN PATENT SPECIFICATION**

(45) Date of publication and mention of the grant of the patent:
**22.11.2023   Bulletin 2023/47**

(21) Application number: **18922817.4**

(22) Date of filing: **30.10.2018**

(51) International Patent Classification (IPC):
*A61K 9/08* (2006.01)      *A61K 38/47* (2006.01)
*A61K 31/7012* (2006.01)      *A61P 27/04* (2006.01)
*A61K 9/00* (2006.01)      *A61K 31/728* (2006.01)
*A61K 47/02* (2006.01)      *A61P 27/02* (2006.01)
*A61P 31/02* (2006.01)      *A61K 47/36* (2006.01)

(52) Cooperative Patent Classification (CPC):
**C12Y 302/01017; A61K 9/0048; A61K 31/728;
A61K 38/47; A61K 47/02; A61K 47/36;
A61P 27/04; A61P 31/02;** Y02A 50/30

(86) International application number:
**PCT/CN2018/112518**

(87) International publication number:
**WO 2019/237634 (19.12.2019 Gazette 2019/51)**

(54) **NOVEL ARTIFICIAL TEARS CONTAINING RECOMBINANT HUMAN LYSOZYME**

NEUARTIGE KÜNSTLICHE TRÄNEN MIT REKOMBINANTEM MENSCHLICHEM LYSOZYM

NOUVELLES LARMES ARTIFICIELLES CONTENANT UN LYSOZYME HUMAIN RECOMBINANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **14.06.2018   CN 201810614434**

(43) Date of publication of application:
**16.06.2021   Bulletin 2021/24**

(73) Proprietor: **Shaanxi Huikang Bio-Tech Co., Ltd.
Xi'an City, Shaanxi Province 710077 (CN)**

(72) Inventors:
• **SHI, Jin
  Yanta District
  Xi'an City, Shaanxi 710077 (CN)**
• **HOU, Zengmiao
  Yanta District
  Xi'an City, Shaanxi 710077 (CN)**
• **GAO, En
  Yanta District
  Xi'an City, Shaanxi 710077 (CN)**
• **LI, Xiaoying
  Yanta District
  Xi'an City, Shaanxi 710077 (CN)**

• **YANG, Xiaolin
  Yanta District
  Xi'an City, Shaanxi 710077 (CN)**
• **ZHAO, Jinli
  Yanta District
  Xi'an City, Shaanxi 710077 (CN)**

(74) Representative: **Habermann, Hruschka &
Schnabel
Patentanwälte
Montgelasstraße 2
81679 München (DE)**

(56) References cited:
**CN-A- 102 188 695      CN-A- 102 552 889
CN-A- 106 265 720      CN-B- 103 182 074
CN-C- 1 193 768      US-A1- 2003 008 805
US-A1- 2007 280 924      US-B2- 7 655 698**

• **Anonymous: "Sodium hyaluronate | 9067-32-7",
Chemical Book, 1 January 2017 (2017-01-01),
pages 1-6, XP055753401, Retrieved from the
Internet:
URL:https://www.chemicalbook.com/ChemicalP
roductProperty_EN_CB4176691.htm [retrieved
on 2020-11-24]**

EP 3 834 839 B1

- **SAAEHA RAUZ ET AL: "Serum eye drops, amniotic membrane and limbal epithelial stem cells-tools in the treatment of ocular surface disease", CELL AND TISSUE BANKING, KLUWER ACADEMIC PUBLISHERS, DO, vol. 11, no. 1, 22 April 2009 (2009-04-22) , pages 13-27, XP019767483, ISSN: 1573-6814**

Remarks:

The complete document including Reference Table(s) and the Sequence Listing(s) can be downloaded from the EPO website

Sequence Listing

<110> SHAANXI IIK BIOTECH C0. LTD
<120> Novel Artificial Tears Containing Recombinant Human Lysozyme
<130> GAI18CN3410P-EP
<160> 4

<170> PatentIn version 3.5
<210> 1
<211> 130
<212> PRT
<213> Human lysozyme
<400> 1

```
        Lys Val Phe Glu Arg Cys Glu Leu Ala Arg Thr Leu Lys Arg Leu Gly
        1               5                   10                  15

        Met Asp Gly Tyr Arg Gly Ile Ser Leu Ala Asn Trp Met Cys Leu Ala
                    20                  25                  30

        Lys Trp Glu Ser Gly Tyr Asn Thr Arg Ala Thr Asn Tyr Asn Ala Gly
                35                  40                  45

        Asp Arg Ser Thr Asp Tyr Gly Ile Phe Gln Ile Asn Ser Arg Tyr Trp
                50                  55                  60

        Cys Asn Asp Gly Lys Thr Pro Gly Ala Val Asn Ala Cys His Leu Ser
        65                  70                  75                  80

        Cys Ser Ala Leu Leu Gln Asp Asn Ile Ala Asp Ala Val Ala Cys Ala
                    85                  90                  95

        Lys Arg Val Val Arg Asp Pro Gln Gly Ile Arg Ala Trp Val Ala Trp
                    100                 105                 110

        Arg Asn Arg Cys Gln Asn Arg Asp Val Arg Gln Tyr Val Gln Gly Cys
                    115                 120                 125

        Gly Val
            130
```

<210> 2
<211> 411
<212> DNA
<213> Artificial Sequence
<400> 2

```
ctcgagaaaa gaaaggtctt tgaaaggtgt gagttggcca gaactctgaa aagattggga      60

atggatggct acaggggaat cagcctagca aactggatgt gtttggccaa atgggagagt     120

ggttacaaca cacgagctac aaactacaat gctggagaca gaagcactga ttatgggata     180

tttcagatca atagccgcta ctggtgtaat gatggcaaaa ccccaggagc agttaatgcc     240

tgtcatttat cctgcagtgc tttgctgcaa gataacatcg ctgatgctgt agcttgtgca     300

aagagggttg tccgtgatcc acaaggcatt agagcatggg tggcatggag aaatcgttgt     360

caaaacagag atgtccgtca gtatgttcaa ggttgtggag tgtaagaatt c             411
```

<210> 3
<211> 34
<212> DNA
<213> Artificial Sequence
<400> 3
gcctcgagaa aagaaaggtc tttgaaaggt gtga 34
<210> 4
<211> 28
<212> DNA
<213> Artificial Sequence
<400> 4
cggaattctt acactccaca accttgaa 28

## Description

## Technical field

The present invention relates to novel artificial tears containing a recombinant human lysozyme, belonging to the field of medicine technology.

## Background Art

Eye inflammation, dry eyes, and eye fatigue are among the most commonly seen conditions in people's daily life. With increasing work-related stress, day-by-day updatings of electronic products, and prolonged use of computers and smart phones, the chance of having dry eyes and dry eye syndrome has been increasing. According to statistics, in recent years the population of dry-eye patients living in cities has been rapidly growing at a rate of 10% to 20% per year, and the incidence is as high as 30%. Studies haven shown that people facing a computer screen for 9 hours or more every day over a long period would double the chance of having an eye disorder than those who do not; nearsighted people facing a computer for a long period is at an even higher risk of having a dry eye disorder. Normally one's eyes blink about 20 times per minute, and this number decreases to 6 if he/she is using a computer or smart phone because the eyes are very much focused and constantly adjust the focal distance to ensure clear objects to be seen. Prolonged, intensive use of eyes, in combination with various radiations from the screen of electronic devices, would have the eyes seriously irritated, resulting in reduced secretion of tears and degeneration of tear components, and in turn causing ophthalmo xerosis, together with superficial ophthalmo xerosis and uncomfortable conditions such as congestion, eye fatigue, decreased vision, and blurred vision.

Ophthalmo xerosis refers to a general term for various conditions including abnormal quality or quantity of tears or abnormal kinetics of tears caused by any reason, which results in decreased stability of the tear film, accompanied by ocular discomfort and/or characteristic pathological changes in the ocular surface tissue. Ophthalmo xerosis can be clinically graded into mild, moderate, and severe degrees, although domestically and abroad there is not yet a gold standard for its grading. However, in general, the mild degree mainly has subjective symptoms, and examinations thereof are mainly based on signs and enquires about the medical history; the moderate degree has subjective symptoms, and examinations thereof mainly involve examining the breakup time of tear film (BUT), cornea fluorescein staining (CFS), and slight keratoconjunctival epithelial injury; and the severe degree is mainly characterized by a BUT less than 5 seconds, seriously reduced tear secretion, obvious cornea and conjunctiva fluorescein staining, and damaged corneal epithelial cells.

The natural tears of human are a transparent fluid secreted by human organs like lacrimal glands, composed of many substances such as inorganic salts, polysaccharides, proteins and lipids, have barrier, bacteriostatic, bactericidal, and immunomodulatory functions, and play an important role in protecting eyeballs, nourishing the ocular surface tissue, accomplishing the visual function, etc. Current commercially available artificial tear products against tear secretion-deficient ophthalmo xerosis of various origins mainly act to supplement basic fluid and moisturize, and are characterized by simple composition and hypotonicity. Although these products can temporarily relieve eye dryness and discomfort, they also further dilute various tear components that are already at low levels on the xerophthalmic ocular surface, such as inorganic salts, lipid transfer proteins, lysozyme, lactoferrin, and the like in tears, making the tear film on the ocular surface more unstable and further weakening the bactericidal and immunomodulatory functions.

On the other hand, many commercially available artificial tear products against symptoms of eye discomfort such as eye dryness, eye irritation, and eye fatigue contain chemical bacteriostatic agents. Such products work well in short-term use, but long-term exposure to bacteriostatic agents may cause irritations such as allergy and dry eye, damage the cornea or conjunctiva and hamper their healing, and may also cause iatrogenic keratitis, etc. Numerous tests have shown that bacteriostatic agents in ophthalmic solutions have cytotoxic effects, which can affect functions and normal metabolism of cells, and long-term use thereof can damage the ocular surface tissue and lower the tolerance of eyes to these ophthalmic solutions. Some even lead to refractory drug-induced conjunctivitis and drug-induced ophthalmo xerosis. Some clinical investigations have shown that, among patients using ophthalmic solutions containing bacteriostatic agents, those having uncomfortable symptoms such as foreign body sensation, tingling sensation, and burning sensation in the eyes are 2.5 times more, and those having red eyes and dry eyes are over 2 times more, than those having the corresponding symptoms but using ophthalmic solutions free of bacteriostatic agents. After these patients switched to ophthalmic solutions free of bacteriostatic agents, these symptoms were apparently alleviated or reversed. Another investigation shows that because the irritating effect of bacteriostatic agents in ophthalmic solutions reduces the compliance of many patients, the number of eye droppings is actively reduced, thereby affecting the clinical therapeutic effects.

US20070280924A1 disclosed a pharmaceutical preparation suitable for use in the eye, which comprises: (i) a pharmaceutically acceptable carrier suitable for use in the eye; (ii) one or more ingredients selected from factors and agents that promote any one or more of survival, health, cell attachment and normal differentiation of ocular surface epithelial cells and optionally factors and agents to prevent squamous metaplasia; (iii) one or more agents capable of altering the fluid properties of a tear film including at least one agent capable of establishing and/or maintaining a stable tear film and optionally one or more agents selected from opthalmological lubricating agents, viscosity enhancing agents and agents capable of reducing tear film evaporation; the factors and agents in component (ii) and (iii) being synthetic or recombinant or licensed for pharmaceutical use.

US7655698B2 disclosed a use of L-carnitine and/or of one or more alkanoyl L-carnitines or one of their pharmaceutically acceptable salts for the preparation of an ophthalmic physiological supplement or medicament in the form of eye-drops, for the treatment of corneal diseases.

SAAEHA RAUZ ET AL (Serum eye drops, amniotic membrane and limbal epithelial stem cells-tools in the treatment of ocular surface disease, published online: 22 April 2009) disclosed that the advent of serum eye drops, amniotic membrane and limbal stem cell grafts, have transformed the treatment of diseases which result in ocular surface failure, and provided an overview of ocular surface anatomy and failure, and how the use of serum eye drops, amniotic membrane and limbal epithelial stem cell transplantation has influenced ophthalmological practice both historically and in recent years. This review focuses on the rationale for the use of these emerging tools, how they are prepared, clinical applications, limitations, and speculates on future directions.

CN102188695A disclosed an ophthalmic gel composition. Each gram of the composition comprises necessary components of: 1000 to 100000 IU (international unit) of epidermal growth factors (EGF), 0.5 to 3.5 mg of hyaluronate sodium, and rest components of pharmaceutical acceptable carriers. It also disclosed a preparation method and a purpose of the composition.

CN103182074B disclosed an ophthalmic preparation containing lysozyme; specifically, the content of lysozyme in the ophthalmic preparation is 0.001%-25% (w/w), 0.01%-20% (w/w), 0.1%-15% (w/w), 0.3%-10% (w/w) or 0.5%-5% (w/w). The ophthalmic preparation of the invention and the lysozyme therein have good stability, have good antibacterial and/or antiseptic properties, and are also beneficial to long-term storage.

CN1193768C disclosed an artificial tear consists of water, water soluble chitin derivative, pH regulator, penetration pressure regulator and sterilizing agent in weight ratio of 1000 to 1-6 to 4-8 to 5-8 to 0.001-0.3. The invention has the advantages of adoption of water soluble chitin derivative as main material, simple preparation process, good moisture absorbing and moisture maintaining performance, no chemical preservative contained, bacterostasis and bactericidal effect on many kinds of bacteria, relatively high viscosity at low concentration and shearing dilution; accords with the requirement on tear; and has excellent eye membrane protecting effect.

US20030008805A1 disclosed ophthalmic compositions having negligible side effects on the heart which can be obtained by using as the active ingredient an adrenergic beta receptor agonist having a high selectivity toward adrenergic beta2 receptor. These compositions are usable as preventives or therapeutics for xerophthalmic disorder and keratoconjunctival disorder.

CN106265720A disclosed a composite artificial tear and a preparation method thereof. The composite artificial tear uses sodium hyaluronate and a chitosan compound as main effective components. The preparation method is simple. The prepared composite artificial tear has good moisture retention performance, is free of any chemical antiseptic, can inhibit a variety of bacteria and fungi, has long action time and exerts good repairing and protection effect on eyes.

## Summary of Invention

The invention is defined in the claims.

In order to solve the above technical problems, an objective of the present invention is to provide novel artificial tears for treatment of clinically common mild-to-moderate ophthalmo xerosis syndrome relating to visual display terminal.

To achieve the above objective, the present invention provides novel artificial tears containing a recombinant human lysozyme, which comprise main components and an auxiliary material, wherein the main components are a recombinant human lysozyme and sodium hyaluronate, and the contents of the recombinant human lysozyme and the sodium hyaluronate are 0.075%-0.300% and 0.10%-0.30%, respectively, based on the total mass of the novel artificial tears; wherein the auxiliary material comprises a pH stabilizer, and the pH stabilizer is a buffer consisting of disodium hydrogen phosphate and sodium dihydrogen phosphate; the auxiliary material comprises sodium chloride, and based on the total mass of the artificial tears, the content of sodium chloride is 0.70%-0.76% as measured in terms of pure solid sodium chloride; wherein the novel artificial tears have a pH of 6.4 to 6.6 and an osmolality of 285 to 310 mOsmol/kg.

According to an embodiment of the present invention, preferably, in the novel artificial tears, the recombinant human lysozyme has an amino acid sequence completely identical to that of natural human lysozyme. Although use of lysozyme as an ocular agent has been reported, the currently used lysozyme is generally extracted from egg white, i.e., hen egg lysozyme, which is different from human lysozyme in amino acid sequence, is heterogenous to human bodies, and often causes side effects such as drug resistance, immune responses, and allergies when used on human bodies. The recombinant human lysozyme (rhLYZ) used in the present invention can be obtained as white lyophilized powder by microbial fermentation, preferably by fermentation of engineered *Pichia* cells expressing human lysozyme and purification of the expressed lysozyme. The rhLYZ has a molecular weight of 14,700 Da, purity greater than 98%, and an amino acid sequence 100% identical to that of natural human lysozyme, being homogenous to a human body. Its amino acid sequence is shown below (SEQ ID NO: 1):

```
  1   KVFERCELAR   TLKRLGMDGY   RGISLANWMC   LAKWESGYNT   RATNYNAGDR
 51   STDYGIFQIN   SRYWCNDGKT   PGAVNACHLS   CSALLQDNIA   DAVACAKRVV
101   RDPQGIRAWV   AWRNRCQNRD   VRQYVQGCGV
```

According to an embodiment of the invention, preferably, in the novel artificial tears, the recombinant human lysozyme is contained in an amount of 0.150% to 0.300% based on the total mass of the artificial tears.

According to an embodiment of the present invention, preferably, the novel artificial tears have a pH of 6.5.

In the novel artificial tears, the auxiliary material comprises a pH stabilizer; and the pH stabilizer is the buffer consisting of disodium hydrogen phosphate and sodium dihydrogen phosphate, so that the pH of the artificial tears can be stabilized at 6.5 to ensure an optimal antibacterial effect of the artificial tears and stability of the formulation, and meanwhile the pH is closer to the pH of natural tears and does not irritate eyes. Preferably, the disodium hydrogen phosphate is disodium hydrogen phosphate dodecahydrate, and the sodium dihydrogen phosphate is sodium dihydrogen phosphate dihydrate, and more preferably, their mass ratio is 20:9.

The novel artificial tears have an osmolality of 285 to 310 mOsmol/kg, which is isotonic with human tears. Hypertonic solutions in eyes may cause the cornea to dehydrate and aggravate the symptoms of eye dryness; hypotonic solutions may make the corneal tissue cells swell or even rupture; and isotonic solution systems can effectively preserve the normal physiological state of the eye tissue cells and alleviate the symptoms of dry eyes.

In the novel artificial tears, the auxiliary material comprises sodium chloride. In the novel artificial tears according to the present invention, the content of sodium chloride is controlled at 0.70%-0.76% (measured in terms of pure solid sodium chloride), which can simultaneously ensure good solubility, activity and stability of the recombinant human lysozyme in the artificial tears, and an osmolality of the artificial tears of 285-310 mOsmol/kg, which is isotonic with human tears.

According to an embodiment of the present invention, preferably, based on the total amount of the novel artificial tears of 10,000-20,000 parts by weight, the novel artificial tears comprise: 7.5-60.0 parts of a recombinant human lysozyme, 10.0-60.0 parts of sodium hyaluronate, 20.0-40.0 parts of disodium hydrogen phosphate dodecahydrate, 9.0-18.0 parts of sodium dihydrogen phosphate dihydrate, 70.0-152.0 parts of sodium chloride, and 9,841.0-19,755.0 parts of water for injection. More preferably, based on the total amount of the novel artificial tears of 10,000 parts by weight, the novel artificial tears comprise: 15.0-30.0 parts of a recombinant human lysozyme, 10.0-30.0 parts of sodium hyaluronate, 20.0 parts of disodium hydrogen phosphate dodecahydrate, 9.0 parts of sodium dihydrogen phosphate dihydrate, 70.0-75.0 parts of sodium chloride, and 9,841.0-9,871.0 parts of water for injection.

According to an embodiment of the present invention, preferably, the novel artificial tears may have the following specific compositions:

based on the total amount of the novel artificial tears of 10,000 parts by weight, the novel artificial tears comprising: 7.5 parts of a recombinant human lysozyme, 10.0 parts of sodium hyaluronate, 20.0 parts of disodium hydrogen phosphate dodecahydrate, 9.0 parts of sodium dihydrogen phosphate dihydrate, 76.0 parts of sodium chloride, and 9,877.5 parts of water for injection; or

based on the total amount of the novel artificial tears of 10,000 parts by weight, the novel artificial tears comprising: 15.0 parts of a recombinant human lysozyme, 10.0 parts of sodium hyaluronate, 20.0 parts of disodium hydrogen phosphate dodecahydrate, 9.0 parts of sodium dihydrogen phosphate dihydrate, 75.0 parts of sodium chloride, and 9,871.0 parts of water for injection; or

based on the total amount of the novel artificial tears of 10,000 parts by weight, the novel artificial tears comprise: 15.0 parts of a recombinant human lysozyme, 30.0 parts of sodium hyaluronate, 20.0 parts of disodium hydrogen phosphate dodecahydrate, 9.0 parts of sodium dihydrogen phosphate dihydrate, 72.0 parts of sodium chloride, and 9,854.0 parts of water for injection; or

based on the total amount of the novel artificial tears of 20,000 parts by weight, the novel artificial tears comprise: 60.0 parts of a recombinant human lysozyme, 20.0 parts of sodium hyaluronate, 40.0 parts of disodium hydrogen phosphate dodecahydrate, 18.0 parts of sodium dihydrogen phosphate dihydrate, 148.0 parts of sodium chloride, and 19,714.0 parts of water for injection; or

based on the total amount of the novel artificial tears of 20,000 parts by weight, the novel artificial tears comprise: 60.0 parts of a recombinant human lysozyme, 60.0 parts of sodium hyaluronate, 40.0 parts of disodium hydrogen phosphate dodecahydrate, 18.0 parts of sodium dihydrogen phosphate dihydrate, 140.0 parts of sodium chloride, and 19,682.0 parts of water for injection.

In order to address the clinically common mild-to-moderate ophthalmo xerosis syndrome relating to visual display terminal caused by reduced nictations and over-evaporation of tears due to excessive use of electronic devices such as computers and mobile phones during people's working and daily life, the present invention provides novel artificial tears having excellent effects of improving the quality and quantity of tear secretion, alleviating dry eye symptoms, and promoting repair of damaged corneal cells, mainly useful for treatment of clinically common mild-to-moderate ophthalmo xerosis syndrome relating to visual display terminal . The novel artificial tears according to the present invention are sterile, free of any chemical bacteriostatic or preservative, can be isotonic with human tears, have the function of maintaining the physiological environment at the ocular surface and suppressing bacteria, and are closer to human natural tears. The artificial tears can be packaged in daily dose units, with a single dose to be used within 24 hours, in a safe and efficacious manner.

The novel artificial tears provided by the present invention can be prepared by a method comprising the following steps:

(1) adding sodium hyaluronate to water for injection and allowing the sodium hyaluronate to be completely dissolved, to obtain a sodium hyaluronate solution;

(2) adding disodium hydrogen phosphate dodecahydrate, sodium dihydrogen phosphate dihydrate and sodium chloride to the sodium hyaluronate solution and allowing them to be completely dissolved, to obtain an auxiliary solution;

(3) adding lyophilized powder of a recombinant human lysozyme to the auxiliary solution, allowing the lyophilized powder of the recombinant human lysozyme to be completely dissolved, and then adjusting the pH of the solution to 6.5±0.1, to obtain the novel artificial tears;

(4) sterilizing the artificial tears by filtration.

The above method for preparation may be implemented in the following specific steps:

(1) preparation of sodium hyaluronate solution
adding sodium hyaluronate to water for injection in a certain ratio, and allowing the sodium hyaluronate to be completely dissolved under stirring (for example, for about 3 hours) to obtain a sodium hyaluronate solution;

(2) preparation of auxiliary solution
adding disodium hydrogen phosphate dodecahydrate, sodium dihydrogen phosphate dihydrate and sodium chloride to the sodium hyaluronate solution in a certain ratio and allowing them to be completely dissolved under stirring, to obtain an auxiliary solution;

(3) preparation of artificial tears
adding lyophilized powder of a recombinant human lysozyme to the auxiliary solution in a certain ratio, allowing the lyophilized powder of the recombinant human lysozyme to be completely dissolved under stirring, and then adjusting the pH of the solution to 6.5±0.1 (for example, adjusting the pH of the solution with hydrochloric acid at a concentration of 5% (w/w)); wherein the well stirred artificial tears should have an osmolarity of 285-310 mOsmol/kg as measured with a freezing point osmometer;

(4) sterilization by filtration
sterilizing the artificial tears by sterile filtration, preferably with a 0.22 μm sterilizing filter, wherein the filtration operation pressure for the sterile filtration may be controlled at 0.35-0.40 MPa; and transferring the sterile artificial tears after the sterile filtration to a sterilized storage tank;

(5) filling
carrying out filling by a Blow-Fill-Seal (BFS) three-in-one sterile filling technique. Blow-Fill-Seal (BFS) means that in a workshop for sterile eye drop production, plastic particles are compressed and heat-melted in an injection molding machine (at 170-230°C, 350 bar (1 bar=0.1 MPa)) and then made into a plastic container, and the container is filled with a prepared sterile liquid under the protection of the A-level laminar flow by a blow-fill-seal machine and sealed, wherein the filling specification can be determined by a person skilled in the art according to actual needs, for example, the filling specification is 0.8 mL/container; wherein the blow molding of bottles, liquid filling, and formulation sealing are done in one set of continuous sterile production procedures.

In the above steps, all pipes used for material transferring must be pre-sterilized.

Most currently available artificial tears are packaged in large doses, which are repeatedly opened during use, and would easily cause secondary contamination by pathogenic microorganisms such as bacteria during long-term use. Therefore, relevant chemical bacteriostatic agents are added to ensure the quality of products during use. Extensive clinical data and research data have shown that many adverse effects seen in ophthalmic treatment are caused by bacteriostatic agents. It has been confirmed in some research that bacteriostatic agents do cause symptoms such as corneal epithelial cell damage, allergies and eye dryness. In order to ensure the safety and practicability of the artificial tears according to the present invention during use, they can be filled into packages of a daily dose, for example, 0.8 mL/package, which are ready to open upon use, with a single dose to be used within 24 hours. The packages of a daily dose are less likely prone to secondary contamination of the artificial tears during use.

The artificial tears according to the invention are a topical external sterile formulation, in which the recombinant human lysozyme is an active protein, and the prepared raw material and auxiliary material are sterilized by a selected terminal filtration means, and the sterile liquid is filled under sterile conditions. This ensures the artificial tears be a sterile formulation and also ensures the activity of the recombinant human lysozyme, effectively avoiding lysozyme inactivation easily caused by high temperature sterilization.

As compared with the prior art, the technical solution of the present invention has the following beneficial effects: the artificial tears according to the present invention comprise a recombinant human lysozyme and sodium hyaluronate as the main components, do not contain any chemical bacteriostatic agent, do not cause any damage to eyes or allergic reactions during long-term use, and are safe and efficacious.

The present invention effectively combines a recombinant human lysozyme, sodium hyaluronate and an auxiliary material together, and upon external administration to eyes, shows significant therapeutic and ameliorating effects on decreased tear secretion, eye dryness, slight inflammation, and slight keratoconjunctival injury caused by mild-to-moderate ophthalmo xerosis.

**Description of Drawings**

Figures 1a to 1d show the bacteriostatic effect of the artificial tears according to the present invention by the paper sheet diffusion method, wherein Figure 1a shows the bacteriostatic effect against *Staphylococcus aureus,* Figure 1b shows the bacteriostatic effect against *Micrococcus luteus,* Figure 1c shows the bacteriostatic effect against *Bacillus subtilis,* and Figure 1d shows the bacteriostatic effect against *Escherichia coli.*

Figure 2 shows the fern-like crystals of tears of New Zealand rabbits' eye from a surgery.

Figure 3 shows the result of fluorescein sodium staining of corneal epithelium of New Zealand rabbits' eye from a surgery.

Figure 4 shows the morphology of fern-like crystals of tears from each group of New Zealand rabbits after 1-week administration.

Figure 5 shows the morphology of fern-like crystals of tears from each group of New Zealand rabbits after 2-week administration.

Figure 6 shows the morphology of fern-like crystals of tears from each group of New Zealand rabbits after 3-week administration.

Figure 7 shows the morphology of fern-like crystals of tears from each group of New Zealand rabbits after 4-week administration.

Figure 8 shows the result of fluorescein sodium staining of corneal epithelium from each group of New Zealand rabbits after 1-week administration.

Figure 9 shows the result of fluorescein sodium staining of corneal epithelium from each group of New Zealand rabbits after 2-week administration.

Figure 10 shows the result of fluorescein sodium staining of corneal epithelium from each group of New Zealand rabbits after 3-week administration.

Figure 11 shows the result of fluorescein sodium staining of corneal epithelium from each group of New Zealand rabbits after 4-week administration.

Figure 12 shows the microscopic photographs (HE staining, $200\times$) of the histology of corneal epithelium from each group of New Zealand rabbits after 4-week administration.

**Detailed Description of Invention**

In order to provide a better understanding of the technical features, objectives and advantageous effects of the present invention, the technical solutions of the present invention will be described below in details, but the detailed description below should not be construed as limiting the implementable scope of the present invention.

The artificial tears according to the present invention were obtained by the inventors through intensive theoretical and experimental research with creative efforts. Although the artificial tears are not completely identical to natural human tears, they have the basic characteristics of tears, including maintaining the physiological environment at the ocular surface (moistening and moisturizing eyeballs, being isotonic and stable), having a bacteriostatic function (the bacteriostatic test in Example 11 shows a bactericidal effect on Gram-positive bacteria) and a natural origin (being sterile and free of chemical bacteriostatic agent), etc., and can exerts the basic functions of natural tears. In the composition of the artificial tears, the selection and ratio of the components also lead to a good synergistic effect to promote each other.

Firstly, the recombinant human lysozyme, as one of the main components of the artificial tears, is a mucopolysaccharide lyase, which is a non-specific immune factor present in normal body fluid and tissues of a human, and also an important component of the human eye immune defense system, having an antibacterial and anti-inflammatory effect. External dropwise addition of human lysozyme can better protect eyes from bacterial infection and inflammation. Moreover, human lysozyme is an intrinsic substance in natural tears of a human body, and long-term use thereof would not cause any toxic side effects to the human body. The test of pharmacodynamic effects of the artificial tears on a rabbit dry-eye model having excessive evaporation of tears accompanied by corneal epithelial cell damage given in Example 13 shows that the artificial tears according to the present invention have significantly better therapeutic effects than the commercially available artificial tear product containing no recombinant human lysozyme but hyaluronic acid alone. Furthermore, the content of recombinant human lysozyme exhibiting the best effect is from 0.150% to 0.300% by weight.

Secondly, sodium hyaluronate, as another main component of the artificial tears, is a non-Newtonian fluid having good biocompatibility, which increases the viscosity of the medicine while overcoming the disadvantage of difficulty in blinking of eyelids. Moreover, sodium hyaluronate has a good moisturizing and lubricating effect, and can make patients with ophthalmo xerosis feel moisturized, refreshed and comfortable in eyes. Under normal circumstances, hyaluronic acid naturally present on the ocular surface forms a film covering the surface of the corneal epithelium, and the corneal epithelium has specific binding sites for hyaluronic acid. The sodium hyaluronate contained in the artificial tears according to the invention forms a film covering the surface of the corneal epithelium, so that the recombinant human lysozyme can stay at the ocular surface for a long time, exerts a bacteriostatic function, reduces invasion of exogenous pathogenic microorganisms into the corneal epithelial cells, and promotes self-repair of damaged cells. The test of pharmacodynamic effects of the artificial tears on a rabbit dry-eye model having excessive evaporation of tears accompanied by corneal epithelial cell damage given in Example 13 confirms that the artificial tears according to the present invention have significantly better therapeutic effects than the control solution containing no hyaluronic acid but recombinant human lysozyme alone.

Thirdly, during the preparation, purification and test of the recombinant human lysozyme, the inventors have surprisingly found that the activity and stability of the recombinant human lysozyme are in a proportional relationship with the sodium chloride content in the solution. The higher the content of the recombinant human lysozyme in the solution, the higher the proportion of sodium chloride required for complete dissolution. The experimental study on the solubility of recombinant human lysozyme and the content of sodium chloride in a solution in Example 3 confirms that, when the content by weight of recombinant human lysozyme is 0.300%, the dissolution stability of the recombinant human lysozyme decreases and turns into a suspension state, with both content and activity decreased, as the salt concentration decreases in a sodium chloride solution at 0.6% or lower, but the dissolution stability and activity of the recombinant human lysozyme maintain substantially constant in a sodium chloride solution at 0.6% to 6%. In the artificial tears according to the present invention, the content of sodium chloride by weight may be controlled at 0.70%-0.76%, which can simultaneously ensure good solubility, activity and stability of the recombinant human lysozyme in the artificial tears, and also an osmolality of the artificial tears of 285-310 mOsmol/kg, which is isotonic with human tears.

In addition, pH is a very important technical indicator in artificial tears, which is related to the stability, effectiveness and irritation to eyes of ophthalmic formulations. The recombinant human lysozyme as an active ingredient has an isoelectric point of 9.24, and shows bacteriostatic activity at pH 5.0-8.0. The experimental study on the bacteriostatic activity and pH of the recombinant human lysozyme given in Example 4 shows that the pH corresponding to the best bacteriostatic activity is 6.5. The ratio of disodium hydrogen phosphate-sodium dihydrogen phosphate used in the present invention can stabilize the pH of artificial tears at 6.5±0.1, which ensures the conditions for optimal activity of the recombinant human lysozyme, and this pH is different from the isoelectric point of the recombinant human lysozyme 9.24 by more than 2 pH units, which ensures long-term stability of the artificial tears, without causing any irritation to human eyes.

In summary, the good ratio and synergistic action of the components in the artificial tears according to the present invention generally ensure the long-term stability of the artificial tears (the stability test results given in Example 12 indicate that the artificial tears show good stability during long-term storage at 25°C or below) and the best activity of recombinant human lysozyme, improve its bioavailability at the ocular surface, stabilize the physiological environment of the ocular surface, protect the corneal cells, promote repair of damaged cells, and effectively treat and relieve the decreased secretion of tears from eyes, eye dryness, and slight inflammation caused by mild-to-moderate ophthalmo xerosis. The test of pharmacodynamic effects of the artificial tears on a rabbit dry-eye model having excessive evaporation of tears accompanied by corneal epithelial cell damage given in Example 13 shows that the artificial tears according to the present invention have clearly efficacious and significantly better effects of improving the quality and quantity of tear secretion and promoting repair of damaged corneal cells than the currently commercially available product.

Example 1. Preparation of recombinant human lysozyme

1. Construction of strains for recombinant human lysozyme

1.1 Construction of expression vector

According to the sequence of human lysozyme (LYZ) published in GenBank, primers were designed with the Primer Premier 5.0 software (the primer sequences are shown in Table 1), wherein the restriction endonuclease Xho I restriction site CTCGAG and the Kex2 restriction site AAAAGA were introduced in the forward primer, and the restriction endonuclease EcoR I site GAATTC was introduced in the reverse primer. The nucleotide sequences after introduction of the aforementioned sites are as follows (SEQ ID NO: 2):

```
1   CTCGAGAAAA GAAAGGTCTT TGAAAGGTGT GAGTTGGCCA GAACTCTGAA AAGATTGGGA
61  ATGGATGGCT ACAGGGGAAT CAGCCTAGCA AACTGGATGT GTTTGGCCAA ATGGGAGAGT
121 GGTTACAACA CACGAGCTAC AAACTACAAT GCTGGAGACA GAAGCACTGA TTATGGGATA
181 TTTCAGATCA ATAGCCGCTA CTGGTGTAAT GATGGCAAAA CCCCAGGAGC AGTTAATGCC
241 TGTCATTTAT CCTGCAGTGC TTTGCTGCAA GATAACATCG CTGATGCTGT AGCTTGTGCA
301 AAGAGGGTTG TCCGTGATCC ACAAGGCATT AGAGCATGGG TGGCATGGAG AAATCGTTGT
361 CAAAACAGAG ATGTCCGTCA GTATGTTCAA GGTTGTGGAG TGTAAGAATT C
```

Table 1. Primer sequences and annealing temperature

| Primer | Primer sequences (5'→3') | Extension length/bp | Annealing temperature /°C |
|---|---|---|---|
| LYZ-XU | GC*CTCGAG*AAAAGAAAGGTCTTTGAAAGGTGTGA (SEQ ID NO: 3) | 415 | 56 |
| LYZ-EL | CG*GAATTC*TTACACTCCACAACCTTGAA ( SEQ ID NO:4) | | |

The human skin cDNA was used as a template, and the designed LYZ-XU and LYZ-EL were used as primers to perform PCR with an annealing temperature of 56°C. The PCR results showed specific bands at expected positions, and the target bands were recovered with a DNA purification and recovery kit. The recovered DNA products, together with the pPIC9K vector (purchased from Invitrogen), were double digested with Xho I (purchased from Thermo Fisher) and EcoR I (purchased from Thermo Fisher), and the digested products were recovered and ligated with DNA ligase, and transformed into *Escherichia coli.* The plasmid was extracted and identified by sequencing, to obtain a human lysozyme expression vector, which was named pPIC9K-LYZ.

1.2 Electrotransformation of *Pichia pastoris*

10 μg pPIC9K-LYZ plasmid linearized with Sal I endonuclease was mixed with 80 μL of competent *Pichia pastoris* cells, transferred to a 0.2 cm electrotransformation cell pre-cooled on ice, electrically shocked for 4-10 ms, and added to a 1 mL solution of 1 mol/L sorbitol pre-cooled on ice. The cells were thoroughly mixed and plated onto an MD (13.4 g/L amino-free yeast nitrogen source (YNB), 20 g/L glucose (Dextrose), and 0.4 mg/L biotin) medium plate, which was inverted and cultured at 30°C for 3-4 days. Colonies grew on the MD medium plate.

1.3 Screening for recombinants with multiple copies of insertions

The colonies grown on the MD medium plate were inoculated with sterilized toothpicks to YPD (1wt% Yeast Extract, 2wt% polypeptone, and 2wt% Dextrose) plates with a G418 concentration of 1 g/L, 2 g/L, 3 g/L, and 4 g/L, respectively, incubated at 30°C, and screened through shaking flasks to obtain the transformants.

2. Fermentation culture and induced expression of recombinant human lysozyme

2.1 Primary seed culturing

The transformants obtained from screening were inoculated separately into 4 flasks each containing 250 mL of a BMGY medium (1% yeast extract; 2% peptone; 100 mmol/L potassium phosphate solution, pH 6.0; 1.34% YNB (Yeast nitrogen base); 1% glycerol), incubated in a thermostatic shaker at 29°C, 225 rpm for 60-70 h, and then inoculated to a seed tank.

2.2 Secondary seed culturing

The primary seeds were transferred to a seed tank containing 3 L of an FBS medium (containing a mixture of 40 g glycerin, 18.2 g $K_2SO_4$, 26.7 mL $H_3PO_4$, 0.93 g $CaSO_4 \cdot 2H_2O$, 14.9 g $MgSO_4$, and 4.13 g KOH per liter), and cultured at a tank temperature controlled at $29.0 \pm 1.0°C$, a tank pressure controlled at $0.050 \pm 0.010$ MPa, and a pH controlled at 5.0. The aeration and stirring speed during the culturing were adjusted to maintain the dissolved oxygen at about 30%. After 16 hours of secondary seed culturing, dissolved oxygen was observed and found to increase significantly (10% increase within 1.0 min). The secondary seed culturing was completed. The seed culturing period is generally 16-24 hours.

2.3 Fermentation in a 150 L fermenter

After the secondary seed culturing was completed, the seeds were transferred to a 150 L fermenter containing 90 L FBS medium, and were cultured at a culture temperature controlled at $29.0 \pm 1.0°C$, a fermenter pressure controlled at $0.050 \pm 0.010$ MPa, a DO of about 30% controlled by manually adjusting the aeration volume, oxygen volume and rotation speed, and a pH of 5.0. Upon culturing for 15-20 h, the substrates were depleted, and the dissolved oxygen rose sharply, indicating start of the feeding stage. Immediately after the start of the feeding stage, oxygen supply was turned off and the stirring speed was lowered to reduce the DO to about 40%. The automatic feeding system was started, and the initial flow rate of a glycerin solution was 1.7 mL/min (1 s/60 s). The fed-batch rate of the glycerin feed was adjusted according to the actual fermentation state, and a GPE antifoam agent was manually supplemented according to the actually foaming state. After feeding for 20 h, samples were taken to measure the wet cell weight of the fermentation broth. When the wet cell weight of the fermentation broth reached 260 g/L, the glycerin feed was stopped and starvation was started.

After the glycerin feed was stopped, the supply of carbon source was insufficient, and the dissolved oxygen again rose significantly. The aeration volume was adjusted and the rotation speed was lowered to control the DO at 30%-40%, to initiate the starvation stage which was controlled to last for 1.0 h. After the starvation was completed, the automatic feeding system was started, the initial feed flow rate was 1.7 mL/min (1 s/60 s), after 3 hours the flow rate was increased to 3.4 mL/min (2 s/60 s), after 6 hours the flow rate was increased to 5.1 mL/min (3 s/60 s), and after 9 hours the methanol induction stage was started. The fed-batch rate of methanol was increased according to the actual level of DO. The fed-batch rate of methanol was generally controlled at up to 13.6 mL/min (8 s/60 s), and the methanol induction period was generally controlled at 40-48 h. In the methanol induction phase, the DO should be controlled at 20-35% and it should be ensured that the methanol accumulation be not excess. When the induction time reached 40-48 h or the methanol supplement volume reached 20-30 L, the methanol induction phase was completed and the product was removed from the fermenter.

3 Purification of recombinant human lysozyme

3.1 Crude purification of recombinant human lysozyme

The fermentation broth was centrifuged in a decanter centrifuge at 2000 rpm for 90 minutes for solid-liquid separation; the supernatant was collected, in which solid sodium chloride was added and completely dissolved to give a final concentration of 0.8 M-1.0 M, and then passed through a 0.2 $\mu$m hollow fiber membrane filtration system to remove residual yeasts and cell debris, especially the green pigments in the fermentation broth of *Pichia pastoris*. The

0.2 μm filtrate was collected and subjected to phenyl hydrophobic chromatography for fine purification.

3.2 Fine purification of recombinant human lysozyme

3.2.1 Purification by hydrophobic chromatography: Preparation of mobile phase A: 1 M sodium chloride solution; phase B: purified water plus 10% isopropanol, at a pH adjusted to 8.0 with a 0.5 M sodium hydroxide solution. Phase A was used to equilibrate a Phenyl Sepharose 6 FF (GE company, USA) high-flow phenyl hydrophobic sepharose gel chromatography column. When equilibrium was reached and the conductivity was 80 mS/cm, the collected 0.2 μm filtrate was loaded onto the column which was then washed with flowing phase A for about 3 column bed volumes, and when the ultraviolet absorption was reduced to below 500 mV, the column was eluted with phase B and the eluate was collected.
3.2.2 Sample processing: The eluate collected in 3.2.1 was adjusted to pH 7.6-7.8 with a 1 M sodium dihydrogen phosphate solution, and the conductivity was 6.0-7.0 mS/cm.
3.2.3 Purification by CM cation exchange chromatography: preparation of mobile phase A: 20 mM phosphate buffer, pH 7.6-7.8, conductivity 2.0-2.3 mS/cm; mobile phase B: 20 mM phosphate buffer, 1 M NaCl, pH 7.6-7.8. The recombinant human lysozyme sample in 3.2.2 was loaded onto a CM Sepharose FF (GE company, USA) high-flow cation exchange sepharose gel chromatography column. After all the sample was loaded, phase A was used to equilibrate the column for 2-3 column bed volumes, a gradient to 16% phase B was applied to remove impurities, then the gradient was adjusted to 25% phase B to carry out elution, and the eluate was collected as the recombinant human lysozyme.
The eluate was purified by Sephadex G25 (GE Company, USA) gel chromatography, and the solution containing the protein peak was collected and freeze-dried to obtain lyophilized powder of the recombinant human lysozyme with a purity of 98% or more.

Example 2: Measurement of quality indices of recombinant human lysozyme

1. Characteristics

The recombinant human lysozyme prepared in Example 1 was white or off-white amorphous powder; odorless; easily destroyed by alkali; easily dissolved in a 0.6% or higher sodium chloride aqueous solution; and insoluble in acetone or diethyl ether.

2. Identification

(1) about 2 mg of the recombinant human lysozyme prepared in Example 1 was dissolved in 2 drops of added physiological saline, and 5 drops of a 10% sodium hydroxide solution and 1 drop of a 10% copper sulfate solution were added and well mixed, resulting in a magenta appearance.
(2) about 2 mg of the recombinant human lysozyme prepared in Example 1 was added to an acetic acid-sodium acetate buffer (6.7 g of anhydrous sodium acetate was added to about 900 mL water, and dissolved under shaking; the pH was adjusted to 5.4 with acetic acid; the solution was diluted to 1000 mL with water, followed by thorough shaking) to make a solution containing 0.2 mg of lysozyme per 1 mL. According to the spectrophotometric method (*Pharmacopoeia of People's Republic of China,* the 2015 edition, Part III, General rule 0401), the maximum absorption at 280 nm was measured as 0.42 to 0.52.
(3) In the chromatogram recorded as an assay item in the purity analysis, the retention time of the main peak of the test sample solution was consistent with the retention time of the main peak of the control solution.
(4) According to the immunoblotting method (*Pharmacopoeia of People's Republic of China,* the 2015 edition, Part III, General rule 3401), it was positive.

3. Molecular weight

According to the mass spectrometry method (*Pharmacopoeia of People's Republic of China,* the 2015 edition, Part IV, General rule 0431), by MALDI-TOF, the molecular weight of the recombinant human lysozyme was measured as 14700 D $\pm$ 100 D. The molecular weight of the recombinant human lysozyme prepared in Example 1 was 14700 D.

4. Isoelectric point

According to the capillary isoelectric focusing electrophoresis method (*Pharmacopoeia of People's Republic of China,* the 2015 edition, Part III, General rule 0542: capillary electrophoresis method), the isoelectric point of the recombinant human lysozyme was 9.24.

### 5. Peptide map

According to the peptide mapping method (*Pharmacopoeia of People's Republic of China,* the 2015 edition, Part III, General rule 3405: the first method: trypsin lysis-reversed phase high performance liquid chromatography), the peptide map of the sample was consistent with that of the control.

### 6. N-terminal amino acid sequence

The sequence of the 15 amino acids at the N-terminus of the recombinant human lysozyme was: Lys-Val-Phe-Glu-Arg-Cys-Glu-Leu-Ala-Arg-Thr-Leu-Lys-Arg-Leu.

### 7. Residual methanol

According to the gas chromatography (*Pharmacopoeia of People's Republic of China,* the 2015 edition, Part III, General rule 0521: gas chromatography), the methanol content was not higher than 0.002%.

### 8. Residual exogenous DNA

According to the residual exogenous DNA assay method (*Pharmacopoeia of People's Republic of China,* the 2015 edition, Part III, General rule 3407), residual exogenous DNA per 100 $\mu$g protein should not exceed 10 ng.

### 9. Residual host protein

According to the assay method for residual protein from engineered yeast cells (*Pharmacopoeia of People's Republic of China,* the 2015 edition, Part III, General rule 3414), the residual host protein should not be higher than 0.1% of total protein.

### 10. pH

Physiological saline was added to 0.1 g of the recombinant human lysozyme prepared in Example 1 to make 10 mL to dissolve the lysozyme, and the pH was 6.5-8.5 as measured by the pH measuring method (*Pharmacopoeia of People's Republic of China,* the 2015 edition, Part III, General rule 0631).

### 11. Weight loss on drying

0.2 g of the recombinant human lysozyme prepared in Example 1 was assayed according to the moisture measuring method (*Pharmacopoeia of People's Republic of China,* the 2015 edition, Part III, General rule 0832), and the weight loss on drying was not more than 10.0%.

### 12. Heavy metal (Pb)

According to the second method for measuring heavy metal (*Pharmacopoeia of People's Republic of China,* the 2015 edition, Part III, General rule 0821), the heavy metal was not more than 10 parts per million.

### 13. Residue on ignition

0.2 g of the recombinant human lysozyme prepared in Example 1 was assayed according to the method for measuring residue on ignition (*Pharmacopoeia of People's Republic of China,* the 2015 edition, Part III, General rule 0841), and the residue was not more than 4.0%.

### 14. Total protein content

The recombinant human lysozyme prepared in Example 1 was assayed according to the protein content measuring method (*Pharmacopoeia of People's Republic of China,* the 2015 edition, Part III, General rule 0731: the second method: Foline-phenol method), and the total nitrogen content was not less than 90% based on dry samples.

15. Purity

(1) According to the non-reducing SDS-polyacrylamide gel electrophoresis method (*Pharmacopoeia of People's Republic of China,* the 2015 edition, Part III, General rule 0541: the fifth method), the content of the recombinant human lysozyme was $\geq 95.0\%$.

(2) According to the reversed-phase high performance liquid chromatography-the area normalization method (*Pharmacopoeia of People's Republic of China,* the 2015 edition, Part III, General rule 0512), the area of the main peak of the recombinant human lysozyme was not smaller than 95% of the total area according to area normalization, wherein the sample loading amount was not less than 5 $\mu$g, the detection was carried out at a wavelength of 280 nm, and the theoretical plate number was not less than 16000 as calculated based on the chromatographic peaks of the recombinant human lysozyme.

16. Biological Activity - Titer measurement

(1) Reagent preparation: phosphate buffer (0.02 M/L, pH 6.5): 3.115 g of $Na_2HPO_4 \cdot 12H_2O$, 1.763 g of $NaH_2PO_4 \cdot 2H_2O$, and 6.0 g of NaCl were weighed out and added to 800 mL of distilled water to be dissolved, and then made up to 1000 mL.

Substrate suspension: 20 mg of *Micrococcus lysodeikticus* powder was weighed out, added to 0.5 mL of phosphate buffer, and soaked for 1 hour. Then an appropriate amount of phosphate buffer was added such that the absorbance of the suspension measured at 450 nm at 25°C was $0.800 \pm 0.05$ (prepared right before use).

Recombinant human lysozyme solution: 10 mg of recombinant human lysozyme was precisely weighed out, placed in a 5 mL volumetric flask, made up to 5 mL with a phosphate buffer, and diluted to obtain a solution of recombinant human lysozyme at 200 $\mu$g/mL.

(2) Operation

0.1 mL of the recombinant human lysozyme solution at a concentration of 200 $\mu$g/mL was pipetted and added at room temperature 25 °C and pH 6.5 into a cuvette containing 3 mL of substrate suspension. The cuvette was placed in an ultraviolet spectrophotometer, the absorbance was measured at 450 nm, and the titer was calculated. A decrease in absorbance of 0.001 per minute represents one unit of enzyme activity. A sample group and a control group were set up. 3 mL of substrate suspension was precisely measured out at $25\pm0.1°C$, and placed in a 1 cm cuvette. The absorbance was measured at 450 nm, and the readings at zero second were recorded as $A_0$ and $A'_0$. Then 0.1 mL of phosphate buffer and 0.1 mL of the 200 $\mu$g/mL recombinant human lysozyme solution were precisely measured out, added to the cuvette, and quickly mixed, and the time was counted with a stopwatch. At 60 seconds, the absorbance values $A_{60}$ and $A'_{60}$ were read, and the activity titer was calculated with the following equation:

$$\text{Activity titer (U/mg)} = \frac{(A_0 - A_{60}) - (A'_0 - A'_{60})}{W} \times 10^6$$

wherein W ($\mu$g) is the weight of the test sample in the measurement liquid.

The measurement was repeated 3 times, and the average value thereof was used to calculate the unit activity of the recombinant human lysozyme which should be not less than $1.0 \times 10^5$ U/mg.

17. Microbial limit

Total number of bacteria (CFU/g): 0.5 g was taken for measurement according to the microbiological limit measuring method for non-sterile products (*Pharmacopoeia of People's Republic of China,* the 2015 edition, Part III, General rule 1105). The total number of bacteria (CFU/g) <100.

The total number of mould and yeasts (CFU/g): 0.5 g was taken for measurement according to the microbiological limit measuring method for non-sterile products (*Pharmacopoeia of People's Republic of China,* the 2015 edition, Part III, General rule 1105). The total number of mould and yeasts (CFU/g) <10.

18. Endotoxin

According to the endotoxin measuring method (*Pharmacopoeia of People's Republic of China,* the 2015 edition, Part III, General rule 1143), endotoxin per 1 mg lyophilized powder of recombinant human lysozyme was less than 10 EU.

After the examinations, the recombinant human lysozyme prepared in Example 1 met the standards and was a qualified product, and can be further used for preparing artificial tears.

Example 3: Study on the solubility of recombinant human lysozyme and the content of sodium chloride in solution

1. Materials and apparatus

1.1 Test materials: the lyophilized powder of recombinant human lysozyme prepared according to Example 1

1.2 Test apparatus: Ultraviolet spectrophotometer, and an electronic balance with a precision of 0.0001 g.

2. Test method

9 batches of 0.0300 g lyophilized powder of recombinant human lysozyme were precisely weighed out on the electronic balance, and placed in nine 10-mL volumetric flasks, respectively. To the volumetric flasks, water for injection and solutions of sodium chloride at a mass concentration of 0.2%, 0.4%, 0.6 %, 0.8%, 1.0%, 2.0%, 4.0%, and 6.0% were respectively added to the volume mark, to obtain 9 solutions of recombinant human lysozyme at a concentration of 3.0 mg/mL theoretically, which were allowed to stand for 1 hour for full dissolution. The state of the solutions was observed. An appropriate amount of each of the 9 solutions was filtered with a 0.22 $\mu$m filter, an appropriate amount of each of the filtrates was taken for measurement of the protein content and the bacteriostatic activity of the 9 samples, according to the total protein amount measuring method and the biological activity-titer measuring method described in Example 2.

3. Test results

The test results for the solubility of recombinant human lysozyme and the sodium chloride content in the solution are summarized in Table 2. It can be seen from the test data that the solutions having a sodium chloride content below 0.6% (0.4%, 0.2%, and water for injection) were a non-clear transparent liquid, generally in a state of suspension; as the salt concentration decreased, the suspension state of the recombinant human lysozyme increased gradually, with the most apparent suspension state of the recombinant human lysozyme found in pure water. At the same time, the protein content and bacteriostatic activity also showed a decreasing trend, mainly because the recombinant human lysozyme was not fully dissolved in solutions having a sodium chloride content below 0.6%. The lower the sodium chloride content, the lower the solubility. The undissolved proteins were filtered off by passing through a 0.22 $\mu$m filter during measurement, and thus only the dissolved proteins in the filtrate and their corresponding bacteriostatic activity were measured. The results also show that the recombinant human lysozyme may be dissolved in pure water in a small amount, and the solubility is limited. In solutions containing 0.6% or more of sodium chloride, 3.0 mg/mL of recombinant human lysozyme were completely dissolved, and the protein content and bacteriostatic activity were stable and consistent.

Table 2. Summary of the results for the solubility of recombinant human lysozyme and the sodium chloride content in the solution

| | Sodium chloride content in solutions of recombinant human lysozyme (%) | | | | | | | | |
|---|---|---|---|---|---|---|---|---|---|
| | 0 | 0.2 | 0.4 | 0.6 | 0.8 | 1.0 | 2.0 | 4.0 | 6.0 |
| State of solution | Suspension | Suspension | Slight suspension | Clear and transparent | Clear and transparent | Clear and transparent | Clear and transparent | Clear and transparent | Clear and transparent |
| Protein content mg/mL | 0.38 | 1.21 | 2.28 | 3.06 | 3.05 | 3.07 | 3.04 | 3.04 | 3.07 |
| Bacteriostatic activity U/mL | $0.53 \times 10^5$ | $1.59 \times 10^5$ | $3.06 \times 10^5$ | $4.30 \times 10^5$ | $4.28 \times 10^5$ | $4.32 \times 10^5$ | $4.33 \times 10^5$ | $4.30 \times 10^5$ | $4.26 \times 10^5$ |

Example 4: Dependency of the bacteriostatic activity of recombinant human lysozyme on pH

1. Materials and apparatus

1.1 Test materials: the lyophilized powder of recombinant human lysozyme prepared according to Example 1

1.2 Test apparatus: Ultraviolet spectrophotometer, and an electronic balance with a precision of 0.0001 g.

2. Test method

7 batches of 0.0300 g lyophilized powder of recombinant human lysozyme were precisely weighed out on the electronic balance, and placed in seven 10-mL volumetric flasks, respectively. To the volumetric flasks, phosphate buffers at a pH of 5.0, 5.5, 6.0, 6.5, 7.0, 7.5, and 8.0 (solutions of 0.02 M phosphate and 0.10 M sodium chloride) were respectively added to the volume mark, to obtain 7 solutions of recombinant human lysozyme at a concentration of 3.0 mg/mL but different pHs, which were each diluted into solutions of recombinant human lysozyme at 1.5 mg/ml and 0.75 mg/mL with a phosphate buffer at a corresponding pH, so as to obtain solutions of recombinant human lysozyme at three different concentrations, with each concentration corresponding to 7 different pHs. The bacteriostatic activity of the 21 samples was measured according to the biological activity-titer measuring method described in Example 2 (in the measuring method, the phosphate buffers were prepared with disodium hydrogen phosphate and sodium dihydrogen phosphate weighed according to the various pHs in this test).

3. Test results

The results of the bacteriostatic activity of recombinant human lysozyme under different pH conditions are summarized in Table 3. It can be seen from the test data that at the same pH the recombinant human lysozyme contents of 0.75 mg/mL, 1.5 mg/mL, and 3.0 mg/mL proportionally correlated with the bacteriostatic activity; the same content displayed activity at different pHs, with the best bacteriostatic activity of recombinant human lysozyme found at pH 6.5 and less bacteriostatic activity found at pH 6.0. When the pH was higher or lower than 6.5, the antibacterial activity showed a decreasing trend as the pH changed. As the pH increased and approached the isoelectric point of recombinant human lysozyme 9.24, the solubility and stability decreased, and the bacteriostatic activity decreased more significantly.

Table 3. Summary of the results of the bacteriostatic activity of recombinant human lysozyme and pH

| Concentration /pH | Bacteriostatic activity (U/mL) | | | | | | |
|---|---|---|---|---|---|---|---|
| | pH5.0 | pH5.5 | pH6.0 | pH6.5 | pH7.0 | pH7.5 | pH8.0 |
| 3.0mg/mL | $3.68 \times 10^5$ | $3.89 \times 10^5$ | $4.24 \times 10^5$ | $4.40 \times 10^5$ | $4.12 \times 10^5$ | $4.03 \times 10^5$ | $3.11 \times 10^5$ |
| 1.5mg/mL | $1.70 \times 10^5$ | $1.87 \times 10^5$ | $2.04 \times 10^5$ | $2.15 \times 10^5$ | $2.00 \times 10^5$ | $1.89 \times 10^5$ | $1.48 \times 10^5$ |
| 0.75mg/mL | $0.79 \times 10^5$ | $0.85 \times 10^5$ | $0.98 \times 10^5$ | $1.04 \times 10^5$ | $0.93 \times 10^5$ | $0.87 \times 10^5$ | $0.67 \times 10^5$ |

The sources of raw materials used in the following Examples 5 to 9 are as follows:

The recombinant human lysozyme was the recombinant human lysozyme prepared in Example 1; Sodium hyaluronate was purchased from Bloomage Freda Biopharm Co., Ltd., as the artificial tear-grade crude sodium hyaluronate, Chinese medicine approval No. H20113379;
Disodium hydrogen phosphate dodecahydrate, sodium dihydrogen phosphate dihydrate, and sodium chloride were purchased from Sinopharm;
Water for injection was made by the applicant by a conventional method.

Example 5

The artificial tears of this Example had a composition shown in Table 4.
The artificial tears of this Example were prepared by the following method:

(1) weighing out 10.0 g sodium hyaluronate and adding it to 9871.0 g water for injection, stirring for 3 hours to allowing the sodium hyaluronate to be completely dissolved, to obtain a sodium hyaluronate solution for further use;
(2) separately weighing out 20.0 g disodium hydrogen phosphate dodecahydrate, 9.0 g sodium dihydrogen phosphate dehydrate, and 75.0 g sodium chloride, adding them to the sodium hyaluronate solution, and allowing these auxiliary materials to be completely dissolved under stirring, to obtain an auxiliary solution for further use;

(3) weighing out 15.0 g lyophilized powder of the recombinant human lysozyme and adding it to the auxiliary solution, followed by stirring until the lyophilized powder was completely dissolved, and then adjusting the pH of the solution to 6.5±0.1 with 5% (w/w) hydrochloric acid; wherein the well stirred artificial tears should have an osmolarity of 285-310 mOsmol/kg as measured with a freezing point osmometer;

(4) sterilizing the artificial tears by sterile filtration with a 0.22 μm sterilizing filter at a filtration operation pressure of 0.35-0.40 MPa; and then transferring the sterile medicine liquid to a sterilized storage tank;

(5) in a workshop for sterile eye drop production, compressing and heat-melting plastic particles in an injection molding machine (at 170-230°C, 350 bar (1 Bar=0.1 MPa)) and making a plastic container therefrom, and filling the container with the sterile medicine liquid prepared in step (4) under the protection of the A-level laminar flow by a blow-fill-seal machine and sealing the container, wherein the filling specification is 0.8 mL/container; then examining, packaging and storing the filled sterile product.

The artificial tears of this Example were in packages of a daily dose, which are ready to open before use, with a single dose to be used within 24 hours.

Examples 6-9

The artificial tears of Examples 6-9 had compositions shown in Table 4.

The artificial tears of Examples 6-9 were prepared by the same method as in Example 5, expect that the amounts of components were accordingly adjusted according to the compositions shown in Table 4.

Table 4 (unit: g)

|  | Example 5 | Example 6 | Example 7 | Example 8 | Example 9 |
|---|---|---|---|---|---|
| Recombinant human lysozyme | 15.0 | 60.0 | 7.5 | 15.0 | 60.0 |
| Sodium hyaluronate | 10.0 | 60.0 | 10.0 | 30.0 | 20.0 |
| $Na_2HPO_4 \cdot 12H_2O$ | 20.0 | 40.0 | 20.0 | 20.0 | 40.0 |
| $NaH_2PO_4 \cdot 2H_2O$ | 9.0 | 18.0 | 9.0 | 9.0 | 18.0 |
| Sodium chloride | 75.0 | 140.0 | 76.0 | 72.0 | 148.0 |
| Water for injection | 9871.0 | 19682.0 | 9877.5 | 9854.0 | 19714.0 |

Example 10: Measurement of quality indices of artificial tears

1. Appearance

The artificial tears of Examples 5-9 were all colorless clear liquid.

2. Identification

(1) To a 1 ml sample from each Example, 5 drops of a 10% sodium hydroxide solution and 1 drop of a 10% copper sulfate solution were added and well mixed, resulting in a magenta appearance.

(2) A 1 ml sample from each Example was added to an acetic acid-sodium acetate buffer solution (6.7 g of anhydrous sodium acetate was added to about 900 mL water, and dissolved under shaking; the pH was adjusted to 5.4 with acetic acid; the solution was diluted to 1000 mL with water, followed by thorough shaking) to make a solution containing 0.2 mg of lysozyme per 1 mL. According to the spectrophotometric method (*Pharmacopoeia of People's Republic of China,* the 2015 edition, Part III, General rule 0401), the maximum absorption was found at 280 nm and was 0.42 to 0.52.

(3) According to the immunoblotting method *(Pharmacopoeia of People's Republic of China,* the 2015 edition, Part III, General rule 3401), it was positive.

3. pH

A 10 ml sample from each Example was assayed according to the pH measuring method *(Pharmacopoeia of People's Republic of China,* the 2015 edition, Part III, General rule 0631), and the pH was 6.5±0.1.

4. Osmolarity

A 1 ml sample from each Example was assayed in a freezing point osmometer according to the osmolarity measuring method *(Pharmacopoeia of People's Republic of China,* the 2015 edition, Part III, General rule 0632), and the osmolarity was 285-310 mOsmol/kg.

5. Visible foreign bodies

According to the method for examining visible foreign bodies (the first method: lamp test) *(Pharmacopoeia of People's Republic of China,* the 2015 edition, Part III, General rule 0904), the product met the regulations for ophthalmic liquid formulations.

6. Load

The labelled load was 0.8 mL/container, the average load was not less than the labelled load, and the load in each container was not less than the labelled load. According to the method *(Pharmacopoeia of People's Republic of China,* the 2015 edition, Part III, General rule 0105), 10 samples from each Example were taken and their contents were poured into a standard volumetric cylinder, followed by visual examination. Each load was not less than the labelled load.

7. Biological Activity - Titer measurement

(1) Reagent preparation: phosphate buffer (0.02 M/L, pH 6.5): 3.115 g of $Na_2HPO_4 \cdot 12H_2O$, 1.763 g of $NaH_2PO_4 \cdot 2H_2O$, and 6.0 g of NaCl were weighed out and added to 800 mL of distilled water to be dissolved, and then made up to 1000 mL.

Substrate suspension: 20 mg of *Micrococcus lysodeikticus* powder was weighed out, added to 0.5 mL of phosphate buffer, and soaked for 1 hour. Then an appropriate amount of phosphate buffer was added such that the absorbance of the suspension measured at 450 nm at 25°C was 0.800 ± 0.05 (prepared right before use).

Recombinant human lysozyme solution: a sample from each Example was placed in a 5 mL volumetric flask, made up to 5 mL with a phosphate buffer, and diluted to obtain a solution of recombinant human lysozyme at 200 μg/mL.

(2) Operation

0.1 mL of the recombinant human lysozyme solution at a concentration of 200 μg/mL was pipetted and added at room temperature 25°C and pH 6.5 to a cuvette containing 3 mL of substrate suspension. The cuvette was placed in an ultraviolet spectrophotometer, the absorbance was measured at 450 nm, and the titer was calculated. A decrease in absorbance of 0.001 per minute represented one unit of enzyme activity. A sample group and a control group were set up. 3 mL of substrate suspension was precisely measured out at 25±0.1°C, and placed in a 1 cm cuvette. The absorbance was measured at 450 nm, and the readings at zero second were recorded as $A_0$ and $A'_0$. Then 0.1 mL of phosphate buffer and 0.1 mL of the 200 μg/mL recombinant human lysozyme solution were precisely measured out, added to the cuvette, and quickly mixed, and the time was counted with a stopwatch. At 60 seconds, the absorbance values $A_{60}$ and $A'_{60}$ were read, and the activity titer was calculated with the following equation:

$$\text{Activity titer (U/mg)} = \frac{(A_0 - A_{60}) - (A'_0 - A'_{60})}{W} \times 10^6$$

wherein W (μg) is the weight of the test sample in the measurement liquid.

The measurement was repeated 3 times, and the average value thereof was used to calculate the unit activity of the recombinant human lysozyme. The biological activity was 70%-200% of the labeled value.

8. Measurement of the sodium hyaluronate content

(1) Preparation of control solution: 60 mg of a glucuronic acid control dried to a constant weight at 60°C and reduced pressure was precisely weighed out, placed in a 100 mL volumetric flask, dissolved in water for injection and diluted to the mark, followed by thorough shaking; 10 mL was precisely measured out, placed in a 100 mL volumetric flask, diluted to the mark with water for injection, and shaken well.

(2) Preparation of the sample solution: from 1-2 artificial tear containers, 0.7 ml was precisely measured out and placed in a 10 mL volumetric flask, diluted to the mark with water for injection, and shaken well.

(3) Preparation of a standard curve: 0, 0.2, 0.4, 0.6, 0.8, and 1.0 mL control solutions were precisely measured out, each placed in a 25 mL test tube equipped with a stopple, made up to 1.0 ml with water, mixed thoroughly, and cooled in an ice bath. Under continuous shaking, 5.0 mL of 0.025 mol/L borax sulfuric acid solution was slowly added dropwise, and the tube was stoppled, heated in a boiling water bath for 10 minutes (shaking it once during the heating), and cooled rapidly. A 0.2 ml solution of 0.125% carbazole in anhydrous ethanol was added, shaken well, heated in a boiling water bath for 15 minutes (shaking it once during the heating), and cooled. According to the ultraviolet-visible spectrophotometric method (*Pharmacopoeia of People's Republic of China*, the 2015 edition, Part III, General rule 0401), with the 0 ml tube as a blank, the absorbance was measured at 530 nm, and the regression equation was calculated for the corresponding absorbance with the number of μg of glucuronic acid.

(4) Assay: 1 mL of the sample solution was precisely measured out, placed it in a 25 mL test tube equipped with a stopple, and assayed according to the method described in "Preparation of a standard curve" from the "cooled in an ice bath" step. The content of glucuronic acid was calculated by the regression equation, and multiplied by 2.0675 to obtain the content.

The measurement was repeated 3 times, and the average value thereof was used to calculate the content of sodium hyaluronate. The sodium hyaluronate content should be between 90% and 110% of the labeled amount.

9. Sterility examination

After processing by membrane filtration, sterility was examined by the sterility examination method (*Pharmacopoeia of People's Republic of China*, the 2015 edition, Part III, General rule 1101), and it should be sterile.
After the examinations, the artificial tears of Examples 5 to 9 were all qualified products.

Example 11: Experimental study on bacteriostatic activity of artificial tears

1. Materials and methods

1.1 Test materials

1.1.1 Test bacteria: *Staphylococcus aureus, Micrococcus luteus, Bacillus subtilis,* and *Escherichia coli.*

1.1.2 Control: 0.1% sodium hyaluronate eye drops (trade name: Aili); Santen Pharmaceutical Co., Ltd., Noto Factory (Japan) (sub-packaged by Santen Pharmaceutical (China) Co., Ltd.)

Blank control: a formulation prepared according to Example 5 but free of recombinant human lysozyme;
Test sample: artificial tears prepared according to Example 5, the activity of which was $2.19 \times 10^5$ U/mL;
1.1.3 Preparation of yeast cells: LB medium: 0.5 g of yeast extract, 1.0 g of peptone, and 1.0 g of sodium chloride were weighed out and dissolved in 100 mL of deionized water, and sterilized at 121°C for 30 minutes; the test yeast cells were inoculated to the LB medium, and incubated overnight at 37°C and 200 rpm.
1.1.4 Preparation of culturing plate: 0.5 g of yeast extract, 1.0 g of peptone, 1.0 g of sodium chloride, and 1.5 g of agar were weighed out and dissolved in 100 mL of deionized water, sterilized at 121°C for 30 minutes, cooled to 50°C, poured onto a sterile watch glass, and naturally cooled to obtain an LB agarose plate ready for use.

2. Test method

100 μL of each test bacterium was uniformly applied to the LB agarose plate, and a piece of round sterile filter paper having a diameter of 6 mm was placed on the coated medium. 5 μL of each of a control, a blank control, and a test sample was dropped on the filter paper, and incubated at 37°C for 24 h. The diameter of the inhibition zone was measured.

3. Results

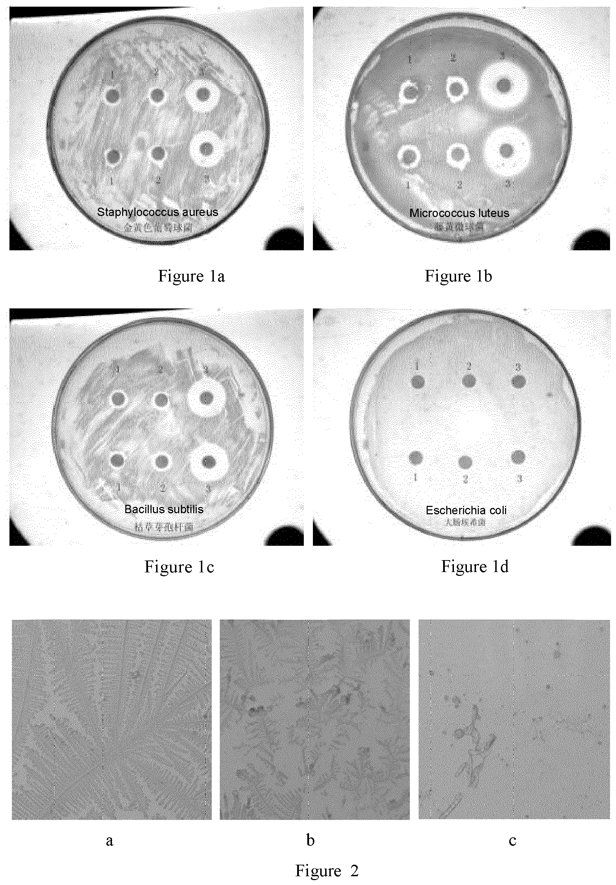

For the bacteriostatic test carried out by paper diffusion bacteriostatic method on the LB agarose plate, the bacteriostatic effect result was shown in Figure 1a to Figure 1d, and summarized in Table 5. It can be seen from the size of the inhibition zone that none of the control, blank control, and test sample has an obvious bacteriostatic effect on the Gram-negative bacterium *Escherichia coli*. As compared with the 0.1% sodium hyaluronate eye drops and the blank control, the artificial tears showed an obvious bacteriostatic effect against Gram-positive bacteria *Staphylococcus aureus, Micrococcus luteus* and *Bacillus subtilis.*

Table 5. Bacteriostatic effect of artificial tears by the paper diffusion method: inhibition zone diameter in mm)

| No. | Name | *Staphylococcus aureus* | *Micrococcus luteus* | *Bacillus subtilis* | *Escherichia coli* |
|-----|------|------|------|------|------|
| 1 | Sodium hyaluronate eye drops (0.1%) | 7 | 9 | 7 | 0 |
| 2 | Blank control | 7 | 9 | 7 | 0 |
| 3 | Recombinant human lysozyme eye drops (0.15%) | 13 | 21 | 16 | 0 |

Example 12: Long-term stability and accelerated test of artificial tears

The artificial tears were tested for stability under the storage conditions prescribed for marketing, and the stability characteristics of the artificial tears during transportation and storage were investigated to provide a basis for determining the period of validity and storage conditions.

The artificial tear sample prepared in Example 5, having an activity of $2.19 \times 10^5$ U/mL, was separately stored at 4°C, 25°C, and 37° C in darkness at humidity of 45%-65%. Samples were taken regularly for examination of the appearance, pH, osmolarity, sterility, and activity. Samples for the long-term stability test were tested once a month for the first 6 months and then once every 3 months for the next 6 months in the first year, and tested once every 6 months in the second year. Samples for the 37°C accelerated test were tested once a month. The results of the stability tests at 4°C, 25°C and 37°C are summarized in Table 6, Table 7 and Table 8, respectively.

Table 6. Summary of the results of stability test of artificial tears (4°C)

| Time | Temp. | Appearance | pH | Osmolarity (mOsmol/kg) | Sterility | Activity (U/mL) |
|------|-------|-----------|-----|------------------------|-----------|-----------------|
| 0 month | - | Colorless clear liquid | 6.50 | 299.2 | Sterile | $2.19 \times 10^5$ |
| 1 month | 4°C | Colorless clear liquid | 6.52 | 300.0 | Sterile | $2.16 \times 10^5$ |
| 2 months | 4°C | Colorless clear liquid | 6.52 | 299.2 | Sterile | $2.18 \times 10^5$ |
| 3 months | 4°C | Colorless clear liquid | 6.48 | 298.4 | Sterile | $220 \times 10^5$ |
| 4 months | 4°C | Colorless clear liquid | 6.50 | 300.0 | Sterile | $2.16 \times 10^5$ |
| 5 months | 4°C | Colorless clear liquid | 6.48 | 299.2 | Sterile | $2.19 \times 10^5$ |
| 6 months | 4°C | Colorless clear liquid | 6.48 | 298.4 | Sterile | $221 \times 10^5$ |
| 9 months | 4°C | Colorless clear liquid | 6.50 | 300.0 | Sterile | $2.17 \times 10^5$ |
| 12 months | 4°C | Colorless clear liquid | 6.52 | 300.0 | Sterile | $2.17 \times 10^5$ |
| 18 months | 4°C | Colorless clear liquid | 6.48 | 300.0 | Sterile | $221 \times 10^5$ |
| 24 months | 4°C | Colorless clear liquid | 6.50 | 298.4 | Sterile | $2.18 \times 10^5$ |

Table 7. Summary of the results of stability test of artificial tears (25°C)

| Time | Temp. | Appearance | pH | Osmolarity (mOsmol/kgl | Sterility | Activity (U/mL) |
|---|---|---|---|---|---|---|
| 0 month | - | Colorless clear liquid | 6.50 | 299.2 | Sterile | $2.19 \times 10^5$ |
| 1 month | 25°C | Colorless clear liquid | 6.52 | 301.6 | Sterile | $2.18 \times 10^5$ |
| 2 months | 25°C | Colorless clear liquid | 6.48 | 300.0 | Sterile | $2.18 \times 10^5$ |
| 3 months | 25°C | Colorless clear liquid | 6.48 | 299.2 | Sterile | $2.19 \times 10^5$ |
| 4 months | 25°C | Colorless clear liquid | 6.52 | 300.0 | Sterile | $2.20 \times 10^5$ |
| 5 months | 25°C | Colorless clear liquid | 6.48 | 300.0 | Sterile | $2.19 \times 10^5$ |
| 6 months | 25°C | Colorless clear liquid | 6.48 | 299.2 | Sterile | $2.18 \times 10^5$ |
| 9 months | 25°C | Colorless clear liquid | 6.50 | 300.0 | Sterile | $2.19 \times 10^5$ |
| 12 months | 25°C | Colorless clear liquid | 6.52 | 300.0 | Sterile | $2.20 \times 10^5$ |
| 18 months | 25°C | Colorless clear liquid | 6.52 | 299.2 | Sterile | $2.17 \times 10^5$ |
| 24 months | 25°C | Colorless clear liquid | 6.52 | 300.0 | Sterile | $2.18 \times 10^5$ |

Table 8. Summary of the results of accelerated test of artificial tears (37°C)

| Time | Temp. | Appearance | pH | Osmolarity (mOsmot/kg) | Sterility | Activity (U/mL) |
|---|---|---|---|---|---|---|
| 0 month | - | Colorless clear liquid | 6.50 | 299.2 | Sterile | $2.19 \times 10^5$ |
| 1 month | 37°C | Colorless clear liquid | 6.48 | 300.0 | Sterile | $2.20 \times 10^5$ |
| 2 months | 37°C | Colorless clear liquid | 6.48 | 299.2 | Sterile | $2.18 \times 10^5$ |
| 3 months | 37°C | Colorless clear liquid | 6.50 | 298.4 | Sterile | $2.19 \times 10^5$ |
| 4 months | 37°C | Colorless clear liquid | 6.52 | 300.0 | Sterile | $2.17 \times 10^5$ |
| 5 months | 37°C | Colorless clear liquid | 6.48 | 299.2 | Sterile | $2.18 \times 10^5$ |
| 6 months | 37°C | Colorless clear liquid | 6.52 | 298.4 | Sterile | $2.17 \times 10^5$ |
| 7 months | 37°C | Colorless clear liquid | 6.50 | 301.6 | Sterile | $2.16 \times 10^5$ |
| 8 months | 37°C | Colorless clear liquid | 6.48 | 305.0 | Sterile | $2.08 \times 10^5$ |
| 9 months | 37°C | Colorless clear liquid | 6.48 | 308.0 | Sterile | $1.78 \times 10^5$ |
| 10 months | 37°C | Colorless clear liquid | 6.48 | 310.2 | Sterile | $1.56 \times 10^5$ |
| 11 months | 37°C | Colorless clear liquid | 6.46 | 322.6 | Sterile | $1.03 \times 10^5$ |
| 12 months | 37°C | Colorless clear liquid | 6.46 | 345.6 | Sterile | $1.01 \times 10^5$ |

RESULTS: The results of the 24-month long-term stability test show that the samples placed at 4°C and 25°C for 24 months exhibited no significant difference in various parameters as compared to the 0 month sample; the results of the accelerated test show that the samples placed at 37°C for up to 12 months exhibited decreased activity of the recombinant human lysozyme and increased osmolarity from the 8th month on, as compared to the 0 month sample; and the sample placed for 11 months was not qualified. Therefore, the artificial tear sample prepared in Example 5 had good stability at 25°C or lower, and under such conditions, the production, transportation and long-term storage will not adversely affect the quality of the product, and can ensure the safety and efficacy for clinical medication.

Example 13: Test of pharmacodynamic effects of artificial tears on a rabbit dry-eye model having excessive evaporation of tears accompanied by corneal epithelial cell damage

1. Materials and methods

1.1 Test sample

1.1.1 Recombinant human lysozyme (rhLYZ) control

Name: 1.5 mg/mL recombinant human lysozyme solution (prepared according to Example 5, free of sodium hyaluronate)
Content: recombinant human lysozyme: 1.5 mg/mL; sodium hyaluronate: 0 mg/mL;
Specification: 0.8 mL: 1.2 mg: 120000 U
Appearance: colorless transparent clear liquid
Storage condition: stored at room temperature (25°C or lower).

1.1.2 Artificial tears containing 0.075% recombinant human lysozyme

Name: Artificial tears of Example 7 (the low-dose group)
Content: recombinant human lysozyme: 0.75 mg/mL; sodium hyaluronate: 1.0 mg/mL;
Specification: 0.8 mL: 0.6 mg: 60000 U
Appearance: colorless transparent clear liquid
Storage condition: stored at room temperature (25°C or lower).

1.1.3 Artificial tears containing 0.15% recombinant human lysozyme

Name: Artificial tears of Example 5 (the middle-dose group)
Content: recombinant human lysozyme: 1.5 mg/mL; sodium hyaluronate: 1.0 mg/mL;
Specification: 0.8 mL: 1.2 mg: 120000 U
Appearance: colorless transparent clear liquid
Storage condition: stored at room temperature (25°C or lower).

1.1.4 Artificial tears containing 0.30% recombinant human lysozyme

Name: Artificial tears of Example 9 (the high-dose group)
Content: recombinant human lysozyme: 3.0 mg/mL; sodium hyaluronate: 1.0 mg/mL;
Specification: 0.8 mL: 2.4 mg: 240000 U
Appearance: colorless transparent clear liquid
Storage condition: stored at room temperature (25°C or lower).

1.2 Control

Name: Hailu® Sodium Hyaluronate Eye Drops;
Manufacturer: URSAPHARM Arzneimittel GmbH (Germany)
Content: 0.1%; that is, 1.0 mg/mL sodium hyaluronate (NaHA);
Specifications: 10 mL/bottle
Appearance: colorless transparent clear liquid
Storage condition: stored at room temperature (25°C or lower).

1.3 About the test sample and the control

The test samples and the control provided were used directly without formulation.

2. Test system

2.1 Animal use management and protection

The National Beijing Center for Evaluation and Research of Drug Safety has received the international certification from AAALAC (Association for Assessment and Accreditation of Laboratory Animal Care International, USA). All animals in the relevant studies according to the present invention were used and managed as required by the international code of ethics and conduct for laboratory animals "Guide for the Care and Use of Laboratory Animals".

2.2 Test system and reasons for selection

Rabbit eyes are convenient for slit lamp microscopy and are a commonly used animal model for studying keratoconjunctivitis sicca (KCS). A common method for establishing a rabbit KCS is to surgically remove the lacrimal gland, the Harderian gland, and the third eyelid, and burn the bulbar conjunctiva of a New Zealand rabbit with 30% trichloroacetic acid.

2.3 Laboratory animals

Species: New Zealand rabbit
Animal grade: ordinary grade
Gender and number: 18 females and 18 males.
Animal weight range: 2-2.5 kg
Animal source: Beijing Xinglong Laboratory Animal Breeding Factory.
Animal license and issuing authority: SCXK (Beijing) 2016-0003, Beijing Municipal Science and Technology Commission.
Animal certificate number: 11805800001595
Animal arrival and reception date: June 23, 2017

2.4 Feeding and management of laboratory animals and their environmental conditions

The room temperature was controlled at 20-25°C and the humidity was 40-70%. 12 h in light and 12 h in darkness. New Zealand rabbits were bred in stainless steel cages, with one animal per cage, and the cages were cleaned once per day.

2.5 Feed and drinking water

Feeds were the standard feeds produced by Beijing Keao Xieli Feed Co., Ltd. (feed certification number: SCXK (Beijing) 2009-0012). The animals were given free access to the feed, and also to pure water which was produced by an SJD animal water dispenser and supplied from drinking water bottles.

2.6 Quarantine process

All animals were subjected to adaptive feeding for one week, during which the animals were observed for their behaviors such as activities and eating/drinking, and no abnormal symptoms were seen. Blood was sampled for hematology examination and no obvious abnormalities were found.

2.7 Method for identification of laboratory animals

With random number method, the animals were grouped with each animal assigned a single laboratory animal number, and excess animals were excluded. The laboratory animal number was used as an identifier in the original data. The cages were labelled with the animal number, dose group and gender.

3. Test design and method

3.1 Establishment and evaluation of dry-eye animal model

3.1.1 Model establishment

The model was established by surgically removing the lacrimal gland, the Harderian gland, and the third eyelid, and burning the bulbar conjunctiva of the New Zealand rabbits with 30% trichloroacetic acid. For each rabbit, the left eye was subjected to the surgery to establish the model, and the right eye served as a control from itself.

3.1.2 Evaluation of the model

36 animals were subjected to a tear secretion test, a tear ferning test, and a slit lamp examination of fluorescein sodium staining before the surgery, one week after, and two weeks after the surgery.

3.1.2.1 Tear secretion test (Schirmer I test)

A 5 mm $\times$ 35 mm stripe of filter paper was bent by 5 mm at one end, which was placed 1/3 inside the conjunctival sac at the inner side of the rabbit's lower eyelid, with the rest being pendent from the skin surface. The rabbit's eyes were gently closed. After 5 minutes, the length of the portion of the filter paper that was wetted with tears was measured. A length below 10 mm/5 min indicated low secretion, and below 5 mm/5 min indicated a dry eye.

3.1.2.2 Tear ferning test (TFT)

Each TFT operation was performed by the same person, and the collecting of tears and the smear operation were performed by a double-blind method. The examination was conducted from 10:00 to 12:00 every day. Tear specimens were drawn from the lacrimal caruncle of the lower eyelid conjunctival sac by siphoning with a glass capillary having an inner diameter of 0.5 mm, and during the operation contact with the ocular surface was avoided. The tear specimens were blown onto a slide, with the airflow kept uniform to avoid generation of bubbles which would affect the crystallization result, then dried at room temperature 20°C for 20 min, and observed under a binocular optical microscope (100$\times$) and graded. By the figure grading method in Rolando *et al.,* the fern-like crystals of tears were classified into four grades according to their integrity, uniformity and branching state.

3.1.2.3 Corneal fluorescein staining (FL)

A 100 g/L fluorescein sodium solution was dropped into the conjunctival sac, which was examined under a slit lamp with cobalt blue light to observe the staining of corneal epithelium. The scoring method was a 12-point method with reference to the expert consensus standards for clinical diagnosis and treatment of ophthalmo xerosis (2013): the cornea was divided into 4 quadrants, with each quadrant given a score of 0-3 points, in which a quadrant having no staining was given 0 point, a quadrant having 1-30 stained spots was given 1 point, a quadrant having more than 30 stained spots without merging of staining was given 2 points, and a quadrant showing merging of stained spots, filament-like bodies and ulcers in the cornea was given 3 points.

3.2 Evaluation of efficacy of artificial tears

3.2.1 Grouping

After successful modeling, the animals were randomized into six groups: a blank control group; a recombinant human lysozyme control group; an artificial tear low-dose group (0.75 mg/mL); an artificial tear middle-dose group (1.50 mg/mL); an artificial tear high-dose group (3.00 mg/mL); and a sodium hyaluronate eye drop control group, with 3 male and 3 female per group, 36 animals in total.

3.2.2 Administration of test samples

    Route: Instillation inside the orbits
    Frequency: administered at 8:00, 12:00 and 16:00 every day, 3 times per day in total.

Period: continuous administration for 4 weeks.
Volume: 2 drops (about 70 µl) / eye.

### 3.2.3 Examination indicators

During the 4-week continuous administration, the tear secretion test, the tear ferning test, and the slit-lamp examination of fluorescein sodium staining were conducted (by the same methods as described above) before the administration, and one week, two weeks, three weeks, and four weeks after the start of administration. At the end of the experiment, the cornea and conjunctiva were taken for HE staining, and histological observation was performed under a microscope.

### 3.3 Statistical analysis

The average value and standard deviation of the measurement data were calculated for each group, and then analyzed by ANOVA.

### 4. Results

### 4.1 Establishment and evaluation of animal models with tear secretion deficiency

### 4.1.1 Tear secretion test (Schirmer I test)

There was no significant difference in the tear-wetted length of the filter paper between the pre-surgery eye and the control eye. One week and two weeks after the surgery, the tear-wetted length of the filter paper in the surgery-post eye was significantly shortened, and highly significantly different from that of the control eye ($p < 0.01$) (the results are shown in Table 9).

### 4.1.2 Tear ferning test (TFT)

According to the grading standard, there was no significant difference in the grade between the pre-surgery eye and the control eye. One week and two weeks after the surgery, the grade of the surgery-post eye increased, and was highly significantly different from that of the control eye ($p<0.01$) (the results are shown in Table 10 and FIG. 2; the a-c in FIG. 2 are for pre-surgery, 1 week after the surgery, and 2 weeks after the surgery, respectively).

### 4.1.3 Corneal fluorescein staining (FL)

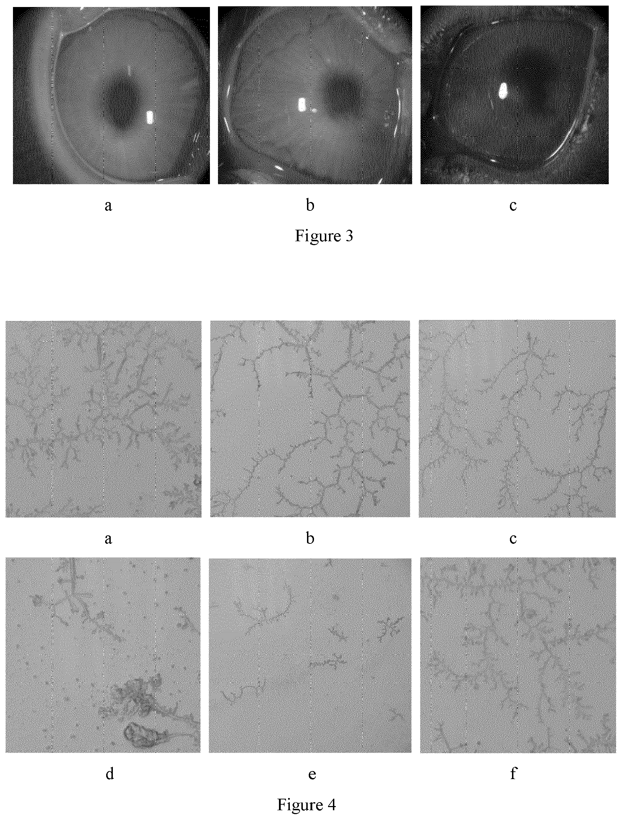

According to the scoring standard, there was no significant difference in the score between the pre-surgery eye and the control eye. One week and two weeks after the surgery, the score of the surgery-post eye increased, and was highly significantly different from that of the control eye ($p<0.01$) (the results are shown in Table 11 and FIG. 3; the a-c in FIG. 3 are for pre-surgery, 1 week after the surgery, and 2 weeks after the surgery, respectively).

### 4.1.4 Summary

Through the tear secretion test, the tear ferning test, and the fluorescein staining examination, the model groups showed a highly significant difference from the control groups, all with statistical significance, indicating that the modeling was successful.

### 4.2 Evaluation of efficacy of artificial tears

### 4.2.1 Tear secretion test (Schirmer I test)

After administration, the tear-wetted length of the filter paper was significantly increased in all of the high-dose group, the middle-dose group, and the low-dose group. Significant difference from the recombinant human lysozyme control group ($p<0.05$) was found at 1, 2, 3 and 4 weeks of administration, with statistical significance. Significant difference from the blank control group ($p<0.05$) was found at 1, 3 and 4 weeks of administration, with statistical significance. Significant difference from the sodium hyaluronate eye drop control group ($p<0.05$) was found at 1 and 4 weeks of administration, with statistical significance (the results are shown in Table 12).

4.2.2 Tear ferning test (TFT)

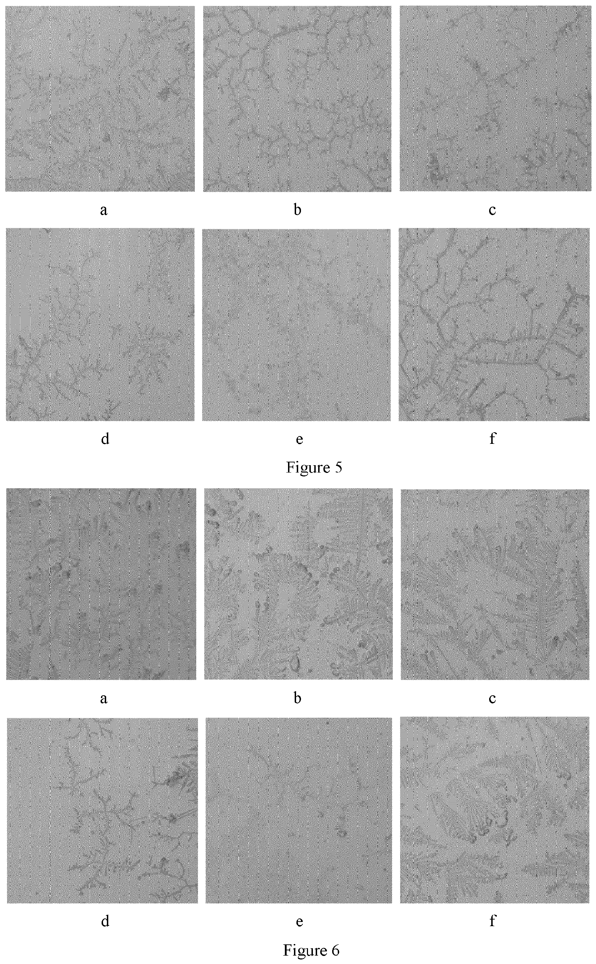

After 1-4 weeks of administration, the high-dose group, the middle-dose group, the low-dose group, and the sodium hyaluronate eye drop control group showed a gradually increased amount of fern-like crystal, significantly different from the blank control group ($p<0.05$), with statistical significance. The recovery degrees of the high- and middle-dose groups were significantly different from that of the sodium hyaluronate eye drop control group were ($p<0.05$), with statistical significance. The recovery degree of the low-dose group was basically the same as that of the sodium hyaluronate eye drop control group. The recovery degree of the recombinant human lysozyme control group was poor and significantly different from that of all dosed groups ($p<0.05$) (the results are shown in Table 13 and Figures 4 to 7; the a-f in Figures 4 to 7 represent the high-dose group, the middle-dose group, the low-dose group, the recombinant human lysozyme control group, the blank control group, and the sodium hyaluronate eye drop control group, respectively).

4.2.3 Corneal fluorescein staining (FL)

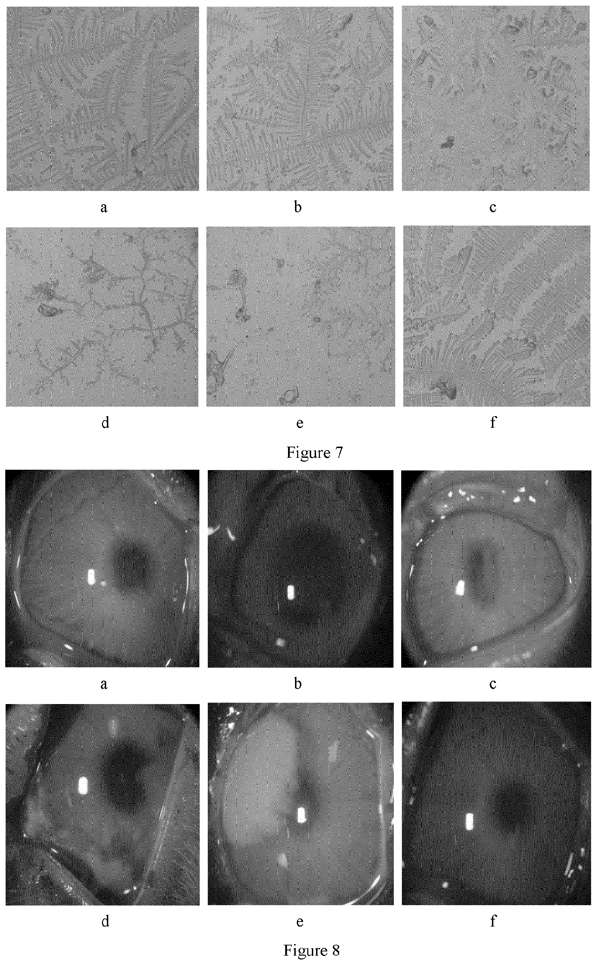

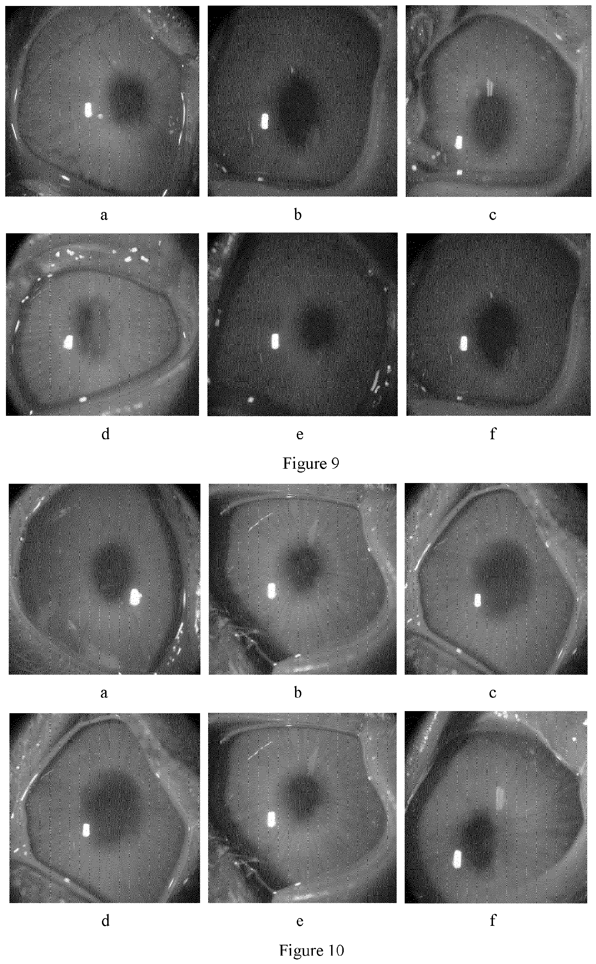

According to the scoring standard, after 1, 2, 3 and 4 weeks of administration, the scores of the high-dose group, the middle-dose group and the low-dose group were significantly different from that of the blank control group ($p<0.05$), with statistical significance; and the scores of the high-dose group, the middle-dose group and the low-dose group were significantly different from that of the recombinant human lysozyme control group ($p<0.05$), with statistical significance. After 3 and 4 weeks of administration, the scores of the high-dose group, the middle-dose group and the low-dose group were significantly different from that of the sodium hyaluronate eye drop control group ($p<0.05$), with statistical significance. In particular, after 2, 3 and 4 weeks of administration, the recovery degrees of the high-dose group and the middle-dose group were significantly different from that of the sodium hyaluronate eye drop control group ($p<0.05$) (the results are shown in Table 14 and Figures 8 to 11; the a-f in Figures 8 to 11 represent the high-dose group, the middle-dose group, the low-dose group, the recombinant human lysozyme control group, the blank control group, and the sodium hyaluronate eye drop control group, respectively).

4.2.4 Histological examination

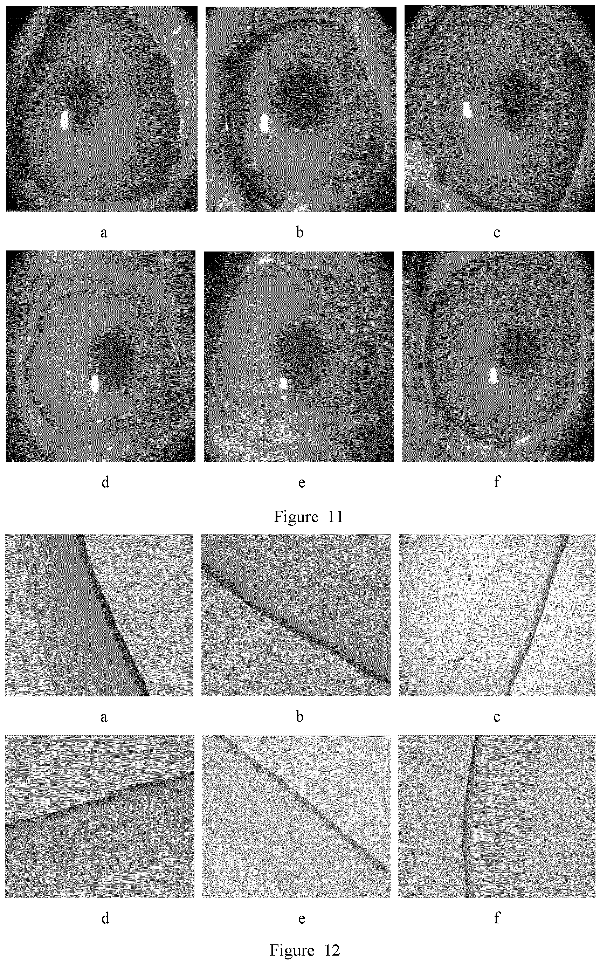

After 4 weeks of treatment and recovery, the corneal epithelium of the New Zealand rabbits was stained with HE and observed under an optical microscope. In the blank control group, the corneal epithelium was thinned and the cell arrangement was disordered. In the low-dose group, the recombinant human lysozyme control group, and the sodium hyaluronate eye drop control group, the corneal epithelium was thinned and the number of cells was small, without a significant difference. In the high-dose and middle-dose groups, the corneal epithelial thickness and cell number recovered well, showing a significant difference from those of the sodium hyaluronate eye drop control group and the recombinant human lysozyme control group (the results are shown in Figure 12; the a-f in Figure 12 represent the high-dose group, the middle-dose group, the low-dose group, the recombinant human lysozyme control group, the blank control group, and the sodium hyaluronate eye drop control group, respectively) .

5 Conclusion

Under the conditions of these tests, as compared with the blank control group, the artificial tears can markedly and effectively improve the quality and quantity of tear secretion, reduce the degree of corneal damage, and protect the corneal epithelium in the tear secretion-deficient ophthalmo xerosis model of New Zealand rabbits; and as compared with the Hailu® sodium hyaluronate eye drop control group and the recombinant human lysozyme control group, the artificial tears, especially in the high-dose group (3.00 mg/mL) and the middle-dose group (1.50 mg/mL), can more effectively improve the quality and quantity of tear secretion, reduce the degree of corneal damage, and promote repair of damaged corneal cells in the tear secretion-deficient ophthalmo xerosis model of New Zealand rabbits.

Table 9. Length (mm) of the portion of filter paper wetted with tears in the surgery-post eye and the control eye

| Group | n | Before Surgery | 1 Week After Surgery | 2 Weeks After Surgery |
|---|---|---|---|---|
| Surgery-post eye | 36 | 19.67±3.70 | 9.94±2.37** | 3.44±1.05** |
| Control eye | 36 | 19.41±3.05 | 19.97±3.00 | 18.46±2.22 |
| Note: Surgery-post eye v.s. Control eye, ** P<0.01 | | | | |

Table 10. Grades from the ferning test of the surgery-post eve and the control eve

| Group | n | Before Surgery | 1 Week After Surgery | 2 Weeks After Surgery |
|---|---|---|---|---|
| Surgery-post eye | 36 | 1.08±0.28 | 2.53±0.91** | 3.53±0.65** |
| Control eye | 36 | 1.11±0.32 | 1.08±0.28 | 1.14±0.35 |
| Note: Surgery-post eye v.s. Control eye, ** P<0.01 | | | | |

Table 11. Scores of fluorescein sodium staining of corneal epithelium on the surgery-post eye and the control eve

| Group | n | Before Surgery | 1 Week After Surgery | 2 Weeks After Surgery |
|---|---|---|---|---|
| Surgery-post eye | 36 | 2.39±1.02 | 6.92±2.49** | 9.52±1.56** |
| Control eye | 36 | 2.52±1.75 | 3.03±1.25 | 3.39±1.24 |
| Note: Surgery-post eye v.s. Control eye, ** P<0.01 | | | | |

Table 12. Secretion test results for artificial tears (mm)

| Group | Before administration | 1-week administration | 2-week administration | 3-week administration | 4-week administration |
|---|---|---|---|---|---|
| High-dose | 3.72±1.36 | 15.33±2.52**#△ | 11.44±2.12△ | 14.44±2.87**△ | 18.22±2.60**#△ |
| Middle-dose | 3.44±0.78 | 13.44±1.82*#△ | 12.44±1.92*#△ | 16.67±2.99**△ | 17.89±2.35**#△ |
| Low-dose | 3.17±0.92 | 14.00±1.91**#△ | 11.78±2.62△ | 15.56±3.68**△ | 17.17±3.09**#△ |
| rhLYZ control | 3.94±1.11 | 6.50±1.58** | 7.39±2.00** | 10.89±3.08 | 11.67±2.52 |
| Blank control | 3.33±0.84 | 11.89±2.19 | 11.11±1.71 | 11.17±2.87 | 10.94±3.42 |
| NaHA eye drop control | 3.00±1.02 | 9.50±2.31** | 10.67±3.29 | 19.61±3.45** | 15.83±3.49** |
| Note: n=6. As compared to the blank control group: *P<0.05, **P<0.01; as compared to the sodium hyaluronate eye drop control group: #p<0.05; as compared to the recombinant human lysozyme control group: △p<0.05. | | | | | |

Table 13. Grades from the ferning test for artificial tears

| Group | Before administration | 1-week administration | 2-week administration | 3-week administration | 4-week administration |
|---|---|---|---|---|---|
| High-dose | 3.67±0.52 | 2.75±0.52*△ | 2.25±0.38*#△ | 1.89±0.23**△ | 1.92±0.22**△ |
| Middle-dose | 3.67±0.51 | 2.88±0.50*△ | 2.38±0.41*#△ | 1.98±0.59*#△ | 1.89±0.37**△ |
| Low-dose | 3.33±0.52 | 2.71±0.54*△ | 2.42±0.62△ | 2.26±0.50△ | 2.02±0.49*△ |
| rhLYZ control | 3.33±0.82 | 3.14±0.39 | 3.09±0.60# | 3.07±0.42*# | 2.31±0.42# |

(continued)

| Group | Before administration | 1-week administration | 2-week administration | 3-week administration | 4-week administration |
|---|---|---|---|---|---|
| Blank control | 3.67±0.52 | 3.70±0.57 | 2.90±0.54 | 2.45±0.26 | 2.56±0.31 |
| NaHA eye drop control | 3.17±0.98 | 2.97±0.32* | 1.94±0.43** | 2.24±0.29 | 1.95±0.35** |
| Note: n=6. As compared to the blank control group: *P<0.05, **P<0.01; as compared to the sodium hyaluronate eye drop control group: #p<0.05; as compared to the recombinant human lysozyme control group: $^{\triangle}$p<0.05. | | | | | |

Table 14. Scores of fluorescein sodium staining of corneal epithelium for artificial tears

| Group | Before administration | 1-week administration | 2-week administration | 3-week administration | 4-week administration |
|---|---|---|---|---|---|
| High-dose | 9.76±1.37 | 7.33±1.75*$^{\triangle}$ | 6.17±1.47**#$^{\triangle}$ | 5.50±1.64*#$^{\triangle}$ | 2.80±1.89*#$^{\triangle}$ |
| Middle-dose | 9.93±2.17 | 8.09±1.01*$^{\triangle}$ | 5.67±1.56**#$^{\triangle}$ | 4.47±1.84*#$^{\triangle}$ | 3.54±2.07*#$^{\triangle}$ |
| Low-dose | 10.13±1.61 | 8.00±2.37*$^{\triangle}$ | 6.50±1.05**$^{\triangle}$ | 5.24±2.36*#$^{\triangle}$ | 3.83±1.33*#$^{\triangle}$ |
| rhLYZ control | 9.35±2.19 | 9.98±2.65 | 8.83±2.57 | 6.39±2.53 | 5.74±2.95 |
| Blank control | 10.65±1.44 | 9.71±2.31 | 9.54±1.24 | 7.88±3.21 | 5.96±2.12 |
| NaHA eye drop control | 10.71±2.05 | 7.67±2.93 | 6.83±1.72 | 6.16±2.32 | 4.85±1.46 |
| Note: n=6. As compared to the blank control group: *P<0.05, **P<0.01; as compared to the sodium hyaluronate eye drop control group: #p<0.05; as compared to the recombinant human lysozyme control group: $^{\triangle}$p<0.05. | | | | | |

Example 14: Test of pharmacodynamic effects of artificial tears on human dry eyes

1. Test materials

Control:

Name: Hailu® Sodium Hyaluronate Eye Drops;
Manufacturer: URSAPHARM Arzneimittel GmbH (Germany)
Content: 0.1%; that is, 1.0 mg/mL sodium hyaluronate (NaHA);
Specifications: 10 mL/bottle
Appearance: colorless transparent clear liquid
Storage condition: stored at room temperature (25°C or lower).
Test Sample: Artificial tears
Name: Artificial tears of Example 5
Content: recombinant human lysozyme: 1.5 mg/mL; sodium hyaluronate: 1.0 mg/mL;
Specification: 0.8 mL: 1.2 mg: 120000 U
Appearance: colorless transparent clear liquid
Storage condition: stored at room temperature (25°C or lower).

2. Test design and method

2.1 Subjects

64 subjects were selected, including 28 males and 36 females, aged 23-50 years. The recruiting criteria were as follows: (1) employees at positions involving long-term working in front of a computer screen with excessive use of eyes, such as financial staff, legal staff, office administrative personnel, lamp inspectors responsible for quality check, and the like; and young employees who use computers and mobile phones for a long time; (2) persons having conscious uncomfortable symptoms in eyes, such as obvious redness and itching in eyes, dry eyes, photophobia, eye fatigue, foreign body sensation, burning sensation, soreness, episodic pain in eyes, and the like; (3) subjects to be excluded: persons having systemic diseases, or having taken glucocorticoids, non-steroidal anti-inflammatory drugs, and immunosuppressive agents.

2.2 Test methods

The 64 subjects were treated with Hailu® sodium hyaluronate eye drops for 14 days, 4 administrations per day, with 2 drops (about 70 $\mu$l) per administration in the left eye; and treated with the artificial tears for 14 days, 4 administrations per day, with 2 drops (about 70 $\mu$l) per administration in the right eye. On the 3rd, 7th, and 14th days since administration, the subjects were observed and the conscious symptoms were recorded.

2.3 Data statistics

The 64 subjects were asked to fill a questionnaire about dry eye symptoms before and after the treatment. The questionnaire included 9 items to be scored, i.e. eye redness, itching, dry eyes, photophobia, eye fatigue, foreign body sensation, burning sensation, soreness, and episodic pain in eyes, and each item was given a score of 0 to 4 from mild to severe in terms of severity and duration in each of the left and right eyes. All data were statistically analyzed using the SPSS20 software. The data were expressed as mean $\pm$ standard deviation, and compared by t-test. $p < 0.05$ was considered statistically significant.

3. Test results

Under the conditions of this test, there were no significant differences in the score of conscious symptoms between the left and right eyes of the 64 subjects before treatment. After 3 days of treatment, the subjects consciously reported a difference in feeling between the left and right eyes, but the data statistics showed no significant difference. After 7 to 14 days of treatment, the subjects consciously reported a marked difference in feeling between the left and right eyes, and the data statistics showed a marked difference between the left and right eyes (the results are shown in Table 15) with statistical significance.

As compared with the Hailu® sodium hyaluronate eye drop control, the tested artificial tears can markedly and more effectively improve the related symptoms of tear secretion-deficient ophthalmo xerosis caused by excessive use of eyes and reduced nictations in people's working and daily life, and promote repair of damaged corneal cells.

Table 15. Summary of scores in the questionnaire about conscious dry eye symptoms

|  | Before treatment | 3-day treatment | 7-day treatment | 14-day treatment |
|---|---|---|---|---|
| Left eye | 18.3±3.46 | 18.6±2.87 | 16.6±3.55 | 13.6±2.76 |
| Right eye | 19.5±2.16 | 15.6±3.75 | 11.7±3.25* | 9.68±4.14* |
| Note: n=64; Right eye compared to left eye, *P<0.05 | | | | |

**Claims**

1. Artificial tears containing a recombinant human lysozyme, said artificial tears comprising main components and an auxiliary material, wherein the main components are a recombinant human lysozyme and sodium hyaluronate, and the contents of the recombinant human lysozyme and the sodium hyaluronate are 0.075%-0.300% and 0.10%-0.30%, respectively, based on the total mass of the novel artificial tears;

   wherein the auxiliary material comprises a pH stabilizer consisting of disodium hydrogen phosphate and sodium dihydrogen phosphate;
   the auxiliary material comprises sodium chloride, and based on the total mass of the artificial tears, the content of sodium chloride is 0.70%-0.76% as measured in terms of pure solid sodium chloride; wherein the novel artificial tears have a pH of 6.4 to 6.6 and an osmolality of 285 to 310 mOsmol/kg.

2. The artificial tears according to claim 1, wherein the recombinant human lysozyme has an amino acid sequence completely identical to that of natural human lysozyme.

3. The artificial tears according to claim 1, wherein the recombinant human lysozyme is contained in an amount of 0.150% to 0.300% based on the total mass of the artificial tears.

4. The artificial tears according to claim 1, wherein the novel artificial tears have a pH of 6.5.

5. The artificial tears according to claim 4, wherein the disodium hydrogen phosphate is disodium hydrogen phosphate dodecahydrate, and the sodium dihydrogen phosphate is sodium dihydrogen phosphate dihydrate.

6. The artificial tears according to claim 5, wherein the mass ratio of disodium hydrogen phosphate dodecahydrate and sodium dihydrogen phosphate dihydrate is 20:9.

7. The artificial tears according to claim 1, wherein based on the total amount of the novel artificial tears of 10,000-20,000 parts by weight, the novel artificial tears comprise: 7.5-60.0 parts of the recombinant human lysozyme, 10.0-60.0 parts of the sodium hyaluronate, 20.0-40.0 parts of d the isodium hydrogen phosphate dodecahydrate, 9.0-18.0 parts of the sodium dihydrogen phosphate dihydrate, 70.0-152.0 parts of the sodium chloride, and 9,841.0-19,755.0 parts of water for injection;

preferably, wherein based on the total amount of the novel artificial tears of 10,000 parts by weight, the novel artificial tears comprise: 15.0-30.0 parts of the recombinant human lysozyme, 10.0-30.0 parts of the sodium hyaluronate, 20.0 parts of the disodium hydrogen phosphate dodecahydrate, 9.0 parts of the sodium dihydrogen phosphate dihydrate, 70.0-75.0 parts of the sodium chloride, and 9,841.0-9,871.0 parts of the water for injection.

8. The artificial tears according to claim 7, wherein based on the total amount of the novel artificial tears of 10,000 parts by weight, the novel artificial tears comprising: 7.5 parts of the recombinant human lysozyme, 10.0 parts of the sodium hyaluronate, 20.0 parts of the disodium hydrogen phosphate dodecahydrate, 9.0 parts of the sodium dihydrogen phosphate dihydrate, 76.0 parts of the sodium chloride, and 9,877.5 parts of the water for injection; or

based on the total amount of the novel artificial tears of 10,000 parts by weight, the novel artificial tears comprising: 15.0 parts of the recombinant human lysozyme, 10.0 parts of the sodium hyaluronate, 20.0 parts of the disodium hydrogen phosphate dodecahydrate, 9.0 parts of the sodium dihydrogen phosphate dihydrate, 75.0 parts of the sodium chloride, and 9,871.0 parts of the water for injection; or

based on the total amount of the novel artificial tears of 10,000 parts by weight, the novel artificial tears comprise: 15.0 parts of the recombinant human lysozyme, 30.0 parts of the sodium hyaluronate, 20.0 parts of the disodium hydrogen phosphate dodecahydrate, 9.0 parts of the sodium dihydrogen phosphate dihydrate, 72.0 parts of the sodium chloride, and 9,854.0 parts of the water for injection; or

based on the total amount of the novel artificial tears of 20,000 parts by weight, the novel artificial tears comprise: 60.0 parts of the recombinant human lysozyme, 20.0 parts of the sodium hyaluronate, 40.0 parts of the disodium hydrogen phosphate dodecahydrate, 18.0 parts of the sodium dihydrogen phosphate dihydrate, 148.0 parts of the sodium chloride, and 19,714.0 parts of the water for injection; or

based on the total amount of the novel artificial tears of 20,000 parts by weight, the novel artificial tears comprise: 60.0 parts of the recombinant human lysozyme, 60.0 parts of the sodium hyaluronate, 40.0 parts of the disodium hydrogen phosphate dodecahydrate, 18.0 parts of the sodium dihydrogen phosphate dihydrate, 140.0 parts of the sodium chloride, and 19,682.0 parts of the water for injection.

**Patentansprüche**

1. Künstliche Tränen, die ein rekombinantes humanes Lysozym enthalten, wobei die künstlichen Tränen Hauptbestandteile und ein Hilfsmaterial umfassen, wobei die Hauptbestandteile ein rekombinantes humanes Lysozym und Natriumhyaluronat sind und die Gehalte des rekombinanten humanen Lysozyms und des Natriumhyaluronats 0,075 % bis 0,300 % bzw. 0,10 % bis 0,30 %, bezogen auf die Gesamtmasse der neuen künstlichen Tränen, betragen;

wobei das Hilfsmaterial einen pH-Stabilisator umfasst, der aus Dinatriumhydrogenphosphat und Natriumdihydrogenphosphat besteht,

das Hilfsmaterial Natriumchlorid umfasst und, bezogen auf die Gesamtmasse der künstlichen Tränen, der Gehalt an Natriumchlorid 0,70 %-0,76 %, gemessen als reines festes Natriumchlorid, beträgt;

wobei die neuen künstlichen Tränen einen pH-Wert von 6,4 bis 6,6 und eine Osmolalität von 285 bis 310 mOsmol/kg aufweisen.

2. Künstliches Tränen nach Anspruch 1, wobei das rekombinante humane Lysozym eine Aminosäuresequenz aufweist, die vollständig mit derjenigen des natürlichen humanen Lysozyms identisch ist.

3. Künstliche Tränen nach Anspruch 1, wobei das rekombinante humane Lysozym in einer Menge von 0,150 % bis 0,300 %, bezogen auf die Gesamtmasse der künstlichen Tränen, enthalten ist.

4. Künstliche Tränen nach Anspruch 1, wobei die neuen künstlichen Tränen einen pH-Wert von 6,5 aufweisen.

5. Künstliche Tränen nach Anspruch 4, wobei das Dinatriumhydrogenphosphat Dinatriumhydrogenphosphat-Dodecahydrat ist und das Nnatriumdihydrogenphosphat Natriumdihydrogenphosphat-Dihydrat ist.

**6.** Künstliche Tränen nach Anspruch 4, wobei das Gewichtsverhältnis von Dinatriumhydrogenphosphat-Dodecahydrat zu Natriumdihydrogenphosphat-Dihydrat 20:9 beträgt.

**7.** Künstliche Tränen nach Anspruch 1, wobei, bezogen auf die Gesamtmenge der neuartigen künstlichen Tränen von 10.000-20.000 Gewichtsteilen, die neuartigen künstlichen Tränen 7,5-60,0 Teile des rekombinanten menschlichen Lysozyms, 10,0-60,0 Teile des Natriumhyaluronats, 20,0-40,0 Teile des Dinatriumhydrogenphosphat-Dodecahydrats, 9,0-18,0 Teile des Natriumdihydrogenphosphat-Dihydrats, 70,0-152,0 Teile des Natriumchlorids und 9.841,0-19.755,0 Teile des Wassers für Injektionszwecke umfassen; vorzugsweise, wobei, bezogen auf die Gesamtmenge der neuen künstlichen Tränen von 10.000 Gewichtsteilen, die neuen künstlichen Tränen 15,0-30,0 Teile des rekombinanten menschlichen Lysozyms, 10,0-30,0 Teile des Natriumhyaluronats, 20,0 Teile des Dinatriumhydrogenphosphat-Dodecahydrats, 9,0 Teile des Natriumdihydrogenphosphat-Dihydrats, 70,0-75,0 Teile des Natriumchlorids und 9.841,0-9.871,0 Teile des Wassers für Injektionszwecke umfassen.

**8.** Künstliche Tränen nach Anspruch 7, wobei, bezogen auf die Gesamtmenge der neuartigen künstlichen Tränen von 10.000 Gewichtsteilen, die neuartigen künstlichen Tränen 7,5 Teile des rekombinanten humanen Lysozyms, 10,0 Teile des Natriumhyaluronats, 20,0 Teile des Dinatriumhydrogenphosphatdodecahydrats, 9,0 Teile des Natriumdihydrogenphosphatdihydrats, 76,0 Teile des Natriumchlorids und 9.877,5 Teile des Wassers für Injektionszwecke umfassen, oder,

bezogen auf die Gesamtmenge der neuartigen künstlichen Tränen von 10.000 Gewichtsteilen, die neuartigen künstlichen Tränen 15,0 Teile des rekombinanten humanen Lysozyms, 10,0 Teile des Natriumhyaluronats, 20,0 Teile des Dinatriumhydrogenphosphatdodecahydrats, 9,0 Teile des Natriumdihydrogenphosphatdihydrats, 75,0 Teile des Natriumchlorids und 9.871,0 Teile des Wassers für Injektionszwecke umfassen, oder,
bezogen auf die Gesamtmenge der neuartigen künstlichen Tränen von 10.000 Gewichtsteilen, die neuartigen künstlichen Tränen 15,0 Teile des rekombinanten humanen Lysozyms, 30,0 Teile des Natriumhyaluronats, 20,0 Teile des Dinatriumhydrogenphosphat-Dodecahydrats, 9,0 Teile des Natriumdihydrogenphosphat-Dihydrats, 72,0 Teile des Natriumchlorids und 9.854,0 Teile des Wassers für Injektionszwecke umfassen, oder,
bezogen auf die Gesamtmenge der neuartigen künstlichen Tränen von 20.000 Gewichtsteilen, die neuartigen künstlichen Tränen 60,0 Teile des rekombinanten humanen Lysozymes, 20,0 Teile des Natriumhyaluronats, 40,0 Teile des Dinatriumhydrogenphosphat-Dodecahydrats, 18,0 Teile des Natriumdihydrogenphosphat-Dihydrats, 148,0 Teile des Natriumchlorids und 19.714,0 Teile des Wassers für Injektionszwecke umfassen, oder,
bezogen auf die Gesamtmenge der neuartigen künstlichen Tränen von 20.000 Gewichtsteilen, die neuartigen künstlichen Tränen 60,0 Teile des rekombinanten humanen Lysozyms, 60,0 Teile des Natriumhyaluronats, 40,0 Teile des Dinatriumhydrogenphosphat-Dodecahydrats, 18,0 Teile des Natriumdihydrogenphosphat-Dihydrats, 140,0 Teile des Natriumchlorids und 19.682,0 Teile des Wassers für Injektionszwecke umfassen.

## Revendications

**1.** larmes artificielles contenant un lysozyme humain recombinant, lesdites larmes artificielles comprenant des composants principaux et un matériau auxiliaire, dans lesquelles les composants principaux sont un lysozyme humain recombinant et un hyaluronate de sodium, et les teneurs en lysozyme humain recombinant et en hyaluronate de sodium sont respectivement de 0,075 % à 0,300 % et de 0,10 % à 0,30 %, sur la base de la masse totale des nouvelles larmes artificielles ;

dans laquelle la matière auxiliaire comprend un stabilisateur de pH constitué d'hydrogénophosphate disodique et de dihydrogénophosphate de sodium ;
la matière auxiliaire comprend du chlorure de sodium, et sur la base de la masse totale des larmes artificielles, la teneur en chlorure de sodium est de 0,70 % à 0,76 %, mesurée en termes de chlorure de sodium solide pur ;
dans lesquelles les nouvelles larmes artificielles ont un pH de 6,4 à 6,6 et une osmolalité de 285 à 310 mOsmol/kg,

**2.** Larmes artificielles selon la revendication 1, dans lesquelles le lysozyme humain recombinant a une séquence d'acides aminés complètement identique à celle du lysozyme humain naturel.

**3.** Larmes artificielles selon la revendication 1, dans lesquelles le lysozyme humain recombinant est contenu dans une quantité de 0,150 % à 0,300 % par rapport à la masse totale des larmes artificielles,

**4.** Larmes artificielles selon la revendication 1, dans lesquelles les nouvelles larmes artificielles ont un pH de 6,5,

5. Larmes artificielles selon la revendication 4, dans lesquelles l'hydrogénophosphate disodique est de l'hydrogénophosphate disodique dodécahydraté, et le dihydrogénophosphate de sodium est du dihydrogénophosphate de sodium dihydraté,

6. Larmes artificielles selon la revendication 5, dans lesquelles le rapport de masse entre l'hydrogénophosphate de sodium dodécahydraté et le dihydrogénophosphate de sodium dihydraté est de 20:9.

7. Les larmes artificielles selon la revendication 1, dans lesquelles, sur la base de la quantité totale des nouvelles larmes artificielles de 10 000-20 000 parties en poids, les nouvelles larmes artificielles comprennent : 7,5-60,0 parties du lysozyme humain recombinant, 10,0-60,0 parties de hyaluronate de sodium, 20,0-40,0 parties d'hydrogénophosphate dodécahydraté d'isodium, 9,0-18,0 parties de dihydrogénophosphate de sodium dihydraté, 70,0-152,0 parties de chlorure de sodium, et 9,841,0-19,755,0 parties d'eau pour injection ;
de préférence, dans laquelle, sur la base de la quantité totale des nouvelles larmes artificielles de 10 000 parties en poids, les nouvelles larmes artificielles comprennent : 15,0-30,0 parties du lysozyme humain recombinant, 10,0-30,0 parties du hyaluronate de sodium, 20,0 parties de l'hydrogénophosphate disodique dodécahydraté, 9,0 parties du dihydrogénophosphate de sodium dihydraté, 70,0-75,0 parties du chlorure de sodium, et 9,841,0-9,871,0 parties de l'eau pour injection.

8. Les larmes artificielles selon la revendication 7, dans lesquelles, sur la base de la quantité totale des nouvelles larmes artificielles de 10 000 parties en poids, les nouvelles larmes artificielles comprennent : 7,5 parties de lysozyme humain recombinant, 10,0 parties de hyaluronate de sodium, 20,0 parties d'hydrogénophosphate disodique dodécahydraté, 9,0 parties de dihydrogénophosphate de sodium dihydraté, 76,0 parties de chlorure de sodium, et 9 877,5 parties d'eau pour injection ; ou

sur la base de la quantité totale des nouvelles larmes artificielles de 10 000 parties en poids, les nouvelles larmes artificielles comprenant : 15,0 parties de lysozyme humain recombinant, 10,0 parties de hyaluronate de sodium, 20,0 parties d'hydrogénophosphate disodique dodécahydraté, 9,0 parties de dihydrogénophosphate de sodium dihydraté, 75,0 parties de chlorure de sodium, et 9 871,0 parties d'eau pour injection ; ou
sur la base d'une quantité totale de larmes artificielles nouvelles de 10 000 parties en poids, les larmes artificielles nouvelles comprennent : 15,0 parties de lysozyme humain recombinant, 30,0 parties de hyaluronate de sodium, 20,0 parties d'hydrogénophosphate disodique dodécahydraté, 9,0 parties de dihydrogénophosphate de sodium dihydraté, 72,0 parties de chlorure de sodium, et 9 854,0 parties d'eau pour injection ; ou
sur la base d'une quantité totale de nouvelles larmes artificielles de 20 000 parties en poids, les nouvelles larmes artificielles comprennent : 60,0 parties de lysozyme humain recombinant, 20,0 parties de hyaluronate de sodium, 40,0 parties d'hydrogénophosphate disodique dodécahydraté, 18,0 parties de dihydrogénophosphate de sodium dihydraté, 148,0 parties de chlorure de sodium, et 19 714,0 parties d'eau pour injection ; ou
sur la base d'une quantité totale de larmes artificielles nouvelles de 20 000 parties en poids, les larmes artificielles nouvelles comprennent : 60,0 parties de lysozyme humain recombinant, 60,0 parties de hyaluronate de sodium, 40,0 parties d'hydrogénophosphate disodique dodécahydraté, 18,0 parties de dihydrogénophosphate de sodium dihydraté, 140,0 parties de chlorure de sodium et 19 682,0 parties d'eau pour préparations injectables.

Figure 1a

Figure 1b

Figure 1c

Figure 1d

a                                    b                                    c

Figure 2

a  b  c

Figure 3

a  b  c

d  e  f

Figure 4

a           b           c

d           e           f

Figure 5

a           b           c

d           e           f

Figure 6

Figure 7

Figure 8

a b c

d e f

Figure 9

a b c

d e f

Figure 10

Figure 11

Figure 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070280924 A1
- US 7655698 B2
- CN 102188695 A
- CN 1193768 C
- US 20030008805 A1
- CN 106265720 A